(19) Europäisches Patentamt / European Patent Office / Office européen des brevets

(11) **EP 3 990 084 B1**

(12) **EUROPEAN PATENT SPECIFICATION**

(45) Date of publication and mention of the grant of the patent:
**24.09.2025 Bulletin 2025/39**

(21) Application number: **20735479.6**

(22) Date of filing: **25.06.2020**

(51) International Patent Classification (IPC):
**A61M 25/00** *(2006.01)* **A61B 5/20** *(2006.01)*

(52) Cooperative Patent Classification (CPC):
**A61M 25/00; A61B 5/205;** A61M 2025/0003;
A61M 2025/0046; A61M 2205/3334; A61M 2205/50;
A61M 2210/1089

(86) International application number:
**PCT/DK2020/050192**

(87) International publication number:
**WO 2020/259782 (30.12.2020 Gazette 2020/53)**

(54) **INTERMITTENT URINARY CATHETER ASSEMBLY COMPRISING SENSOR ELEMENT**

INTERMITTIERENDE HARNKATHETERANORDNUNG MIT SENSORELEMENT

ENSEMBLE SONDE URINAIRE INTERMITTENTE COMPRENANT UN ÉLÉMENT CAPTEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2019 DK PA201970408**

(43) Date of publication of application:
**04.05.2022 Bulletin 2022/18**

(73) Proprietor: **Coloplast A/S**
**3050 Humlebaek (DK)**

(72) Inventor: **PEDERSEN, Troels Gottfried**
**3250 Gilleleje (DK)**

(56) References cited:
**CN-A- 103 263 701        CN-A- 107 981 873**
**CN-U- 201 782 870        CN-U- 206 577 223**
**US-A- 5 476 434          US-A1- 2005 256 447**
**US-A1- 2009 306 539**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to relieving urinary retention and the field of intermittent catheterization.

US2005/0256447 discloses an apparatus for controlling the operation of a catheter including an inlet for receiving a catheter and an outlet for discharging liquid, valve means, sensing means, and control means for controlling operation of the valve means in response to a predetermined criteria.

CN201782870U discloses a urine leading device including a pressure sensor and a temperature sensor for real-time in-body monitoring of pressure and temperature in the bladder.

CN107981873A discloses a urethral canal pressure measuring device.

US2009/0306539A1 discloses a pressure sensing catheter including a catheter shaft, a pressure sensing balloon located on the catheter shaft and a transducer base including a pressure sensor assembly.

CN103263701A discloses a bladder intermittent drainage system.

US5476434 discloses a system for controlling drainage of a female bladder including a valved catheter, a stylet for placing the catheter in the female urethra and a spike for opening the valve in the catheter also including sensing and transmission means. CN206577223U discloses a pressure measurement catheter.

BRIEF SUMMARY

The invention is defined by independent claim 1. Further aspects of the invention are defined in dependent claims 2-22.

Embodiments provide an intermittent urinary catheterisation assembly for self-catheterisation. The intermittent urinary catheterisation system comprises an intermittent urinary catheter comprising a catheter tube. The catheter tube comprises an insertable portion intended for insertion into a user's urethra, a non-insertable portion not intended for insertion into the user's urethra, and a connecting portion being integral with or mounted to the non-insertable portion. The intermittent urinary catheter further comprises a conduit extending longitudinally within the catheter tube and defining at least part of a flow path from a distal insertion end of the catheter to a proximal outlet end thereof. The assembly further comprises a measuring system for determining at least one fluid parameter in the flow path. The measuring system comprises at least one sensor element configured to determine the at least one fluid parameter in the conduit and/or in a space in communication with the conduit and a signal processing device. In one aspect, the signal processing device comprises a housing with an engagement mechanism for detachably securing the signal processing device in relation to the connecting portion of the intermittent urinary catheter, in which case the assembly comprises an interface for connecting the signal processing device to the at least one sensor element. In another aspect, the measuring system is securely connected to or integrated with the non-insertable portion of the catheter tube without the need of a catheter tube connecting portion. An intermittent urinary catheter system and a method of intermittent urinary catheterisation are also disclosed but the method is not part of the claimed invention and is left for illustrative purposes.

BRIEF DESCRIPTION OF THE DRAWINGS

The accompanying drawings are included to provide a further understanding of embodiments and are incorporated in and constitute a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.

Figs. 1-12 illustrate schematic cross-sectional views of different embodiments of an intermittent urinary catheter assembly with an intermittent urinary catheter and a measuring system.

Fig. 13 illustrates a schematic cross-sectional view of an embodied flow restrictor.

Figs. 14-15 illustrate schematic cross-sectional views of embodied measurement substructures and signal processing devices.

Fig. 16 illustrates a schematic cross-sectional view of an embodied intermittent urinary catheter.

Fig. 17 illustrates a plot of measured voiding times for an embodied intermittent urinary catheterisation assembly 1.

DETAILED DESCRIPTION

Intermittent catheters are typically inserted by the user him- or herself and sits only in the urethra and bladder for as long as it takes to empty the bladder - e.g. for about 5-10 minutes. Intermittent catheters are commonly used every 4-6 hours to empty the bladder corresponding roughly to the interval that people having no urinary problems will usually go to the bathroom. Intermittent catheters are typically more rigid than indwelling catheters since they have to be inserted by the user him-/herself and since they do not need to sit in the urethra for days or weeks. An important feature for the intermittent catheter is to ease the insertion into the urethra. This is done by providing the intermittent catheter with a low frictious surface. Non-limiting examples of such are hydrophilic coated catheters which are subsequently wetted by a swelling media in order to produce a low friction surface, or oil or water-based gel which is applied to the catheter before insertion into the urethra.

Intermittent urinary catheters may be provided with a hydrophilic coating that needs to be wetted prior to use and thereby absorbs a considerable amount of liquid. Such a hydrophilic coating will provide a very lubricious surface that has very low-friction when the catheter is to be inserted. Hydrophilic coated catheters, where the coating absorbs a considerable amount of liquid for a low frictious surface (swelling degree >100%), will not be suitable for indwelling catheters, because the hydrophilic surface coating would stick inside the mucosa of the urethra if left inside the body for a longer period, due to the hydrophilic coating transforming from being highly lubricious when fully wetted to being adhesive when the hydration level of the coating is reduced.

This invention relates to intermittent urinary catheters that may be provided with a hydrophilic coating of the kind that is wetted prior to use to absorb a considerable amount of liquid and to provide a very lubricious surface.

Usually catheters used as urinary draining devices are from size 8 FR to size 18 FR. FR (or French size or Charriere (Ch)) is a standard gauge for catheters approximately corresponding to the outer circumference in mm. More accurately, the outer diameter of the catheter in mm corresponds to FR divided by 3. Thus 8 FR corresponds to a catheter with an outer diameter of 2.7 mm and 18 FR corresponds to a catheter with an outer diameter of 6 mm.

Intermittent urinary catheters typically range from CH 8 - CH 16.

Intermittent urinary catheters are predominantly used for self-catheterisation by the user.

Embodiments provide an intermittent urinary catheter assembly and an intermittent urinary catheter that enable measurement of a fluid parameter for increased utility and convenience for the user. The intermittent urinary catheter assembly and intermittent urinary catheter provide a sensor element configured to determine the fluid parameter.

In a first aspect, embodiments provide an intermittent urinary catheterisation assembly for self-catheterisation comprising:

- an intermittent urinary catheter comprising:

  ○ a catheter tube comprising an insertable portion intended for insertion into a user's urethra, a non-insertable portion not intended for insertion into the user's urethra, and a connecting portion being integral with or mounted to the non-insertable portion;
  ○ a conduit extending longitudinally within the catheter tube and defining at least part of a flow path from a distal insertion end of the catheter to a proximal outlet end thereof;

- a measuring system for determining at least one fluid parameter in the flow path, comprising

  ○ at least one sensor element configured to determine the at least one fluid parameter in the conduit and/or in a space in communication with the conduit;
  ○ a signal processing device comprising

    ▪ a housing with an engagement mechanism for detachably securing the signal processing device in relation to the connecting portion of the intermittent urinary catheter; and
    ▪ an interface for connecting the signal processing device to the at least one sensor element.

In a second aspect, embodiments provide an intermittent urinary catheter for use in an intermittent urinary catheterisation assembly according to the first aspect, comprising

- a catheter tube comprising an insertable portion intended for insertion into a user's urethra and a non-insertable portion not intended for insertion into the user's urethra, wherein the non-insertable portion defines a connecting portion integral with or mounted to the non-insertable portion;
- a conduit extending longitudinally within the catheter tube and defining at least part of a flow path from a distal insertion end of the intermittent urinary catheter to a proximal outlet end thereof,

wherein the connecting portion is configured to mate with the engagement mechanism of the assembly.

Thanks to the provision of the at least one sensor element and the signal processing device a user of an embodied intermittent urinary catheter assembly is enabled to determine at least one fluid parameter in the conduit of the intermittent urinary catheter and/or in a space in communication with the conduit. The at least one fluid parameter may be relevant for several aspects of intermittent catheterisation and provide information on, e.g., the fluid flow rate and/or amount of urine voided during intermittent catheterisation, the filing degree of the bladder prior to intermittent catheterisation, the hydration level of the user, the presence and/or concentration of one or more analytes. Accordingly, the user is provided with improved utility of the intermittent urinary catheter assembly and may be able to make informed choices based on the information determined by the at least one sensor element and signal processing device.

At least one of the at least one sensor element may be imbedded in or integral with the catheter tube. Alternatively or additionally at least one of the at least one sensor element may be comprised in the housing of the signal processing device. In each case, the interface allows the signal processing device to establish a data connection with the at least one sensor element internally in the housing of the signal processing device, or externally between the catheter tube and the signal processing device.

In embodiments, the intermittent urinary catheter is disposable.

The connecting portion of the intermittent urinary catheter in conjunction with the engagement mechanism enables detachable securement of the signal processing device in relation to the catheter tube. Accordingly, the intermittent urinary catheter may be disposed of after each intermittent catheterisation, while the signal processing device may be used a plurality of times for more effective use of resources.

In embodiments, when the connecting portion and the engagement mechanism are properly secured in relation to each other fluid communication between the conduit and the at least one sensor element is enabled.

In embodiments, the connecting portion of the catheter tube and the engagement mechanism of the measuring device comprise respective, mutually mating connection elements. In one embodiment, the engagement mechanism comprises resilient members configured to engage and at least partially surround, e.g., grooves at the outer circumference of the connecting portion of the catheter tube, which may provide improved tactile feedback for, e.g., physically impaired users. The engagement mechanism and connecting portion may also be embodied as, e.g., counterparts of a detachable click-lock for ease of manufacture, mutually attracting magnets for ease of attachment due to the attractive forces of the magnets working at a distance, or a frictional coupling where a protruding connecting portion of the catheter tube is forced into a receiving portion of the engagement mechanism or vice versa.

In embodiments, the at least one fluid parameter comprises at least one of: pressure, fluid flow rate, particle size, particle count, particle distribution, particle concentration, particle characteristics, analyte concentration, refractive index, conductivity, temperature, absorption spectrum, refraction spectrum, viscosity, pH. Each parameter may be used to determine information relevant to the user. A sensed pressure and/or fluid flow rate may be used to determine, e.g., a urine fluid flow rate, a pressure in the bladder, an initial pressure in the bladder prior to urine voiding, a measure of the degree of expansion of the bladder, the amount of urine evacuated during intermittent catheterisation, and/or if urine voiding has suddenly stopped. The different particle characteristics allow derivation of information regarding the condition of the user. For instance, certain particle sizes/counts/distributions/concentrations may be indicators of a condition or disease. For example, a relatively high amount of protein in the urine could be signs of damage to the glomeruli. Fluid parameters such as analyte concentration, absorption spectra (colour) and conductivity may be used to determine a hydration level of the user, which in turn may help the user to prevent or alleviate dehydration, which helps to prevent infection of the bladder and/or urinary tract. The particle characteristics and spectra may also be used to establish presence and concentration of cells in the urine such as leukocytes. Any more than trace amounts of leukocytes in the urine may indicate a urinary tract- or bladder infection. Such conditions are particularly relevant to detect for users of intermittent urinary catheters as they are generally relatively exposed to such infections due to the repeated introduction of an intermittent urinary catheter in the urethra.

In embodiments, the signal processing device comprises wired or wireless connection means for connecting to- and transmitting data to another electronic device such as, e.g., a portable electronic device such as a smartphone or laptop, or a stationary computer or server for further data analysis and/or output.

In embodiments, the measuring system comprises data output means. The data output means may comprise a visual output unit possibly with resolution of colour. The visual output unit may be integrated in the housing of the signal processing device or in an external device with data connection to the signal processing device. In embodiments, the data output means comprises tactile feedback means. The tactile feedback means may particularly comprise a vibrator. The tactile feedback is able to provide feedback to the user without requiring visual contact with the intermittent urinary catheterisation assembly. Such feedback is particularly advantageous in relation to intermittent catheterisation as it is commonly done by the user him- or herself and visual inspection of the intermittent catheterisation assembly may be difficult and/or inconvenient. In one embodiment, the measuring system is configured to provide tactile feedback to the user in response to detection of a fluid flow rate, and/or a derivative thereof, below a first threshold in the conduit, and/or provide tactile feedback in response to detection of a fluid flow rate, or a derivative thereof, above a second threshold in the conduit for increased user convenience.

In embodiments, the intermittent urinary catheter is configured to rest in the user's urethra for a period of time not exceeding 15 minutes. This allows the intermittent urinary catheter to comprise, e.g., coatings that do not allow the extended duration of catheterisation inquired for indwelling catheterisation.

In embodiments, at least an outer surface of the insertable portion of the catheter tube comprises a hydrophilic surface coating. Such hydrophilic coating allows particularly convenient intermittent urinary catheterisation.

The hydrophilic coating may be provided only on the insertable part of the catheter. The hydrophilic surface coating is of the kind which, when hydrated or swelled using a swelling medium, reduces the friction on the surface area of the catheter which is intended to be inserted into the urinary channel of a user corresponding to the insertable part of the catheter.

An intermittent hydrophilic catheter differs from an indwelling catheter also in that the hydrophilic surface coating of such a catheter is not suitable for indwelling use, because the surface coating tends to stick inside the mucosa of the urethra if left inside the body for a period exceeding 5-20 minutes, due to the hydrophilic coating transforming from being highly lubricious when fully wetted (95% weight water) to being adhesive when the hydration level of the coating is reduced (<75% weight water).

The catheter tube is a single-lumen tube, of which the conduit constitutes the sole passage between the distal insertion end and the proximal outlet end of the intermittent urinary catheter. Such single-lumen tube catheters are generally not applicable for indwelling use because of the lack of a passage for conducting air to the balloon of an indwelling- or Foley urinary catheter.

In embodiments, at least one of the at least one sensor element is comprised in the signal processing device. This allows the at least one sensor element in the signal processing device to be re-used while the intermittent urinary catheter is conveniently disposed of. In one embodiment, the signal processing device comprises wired or wireless connection means for connecting to- and transmitting data to another electronic device such as, e.g., a portable electronic device such as a smartphone or laptop, or a stationary computer or server. In one embodiment, the signal processing device comprises data output means as those described above.

In embodiments, the measuring system comprises a measurement substructure embedded in or integral with the catheter tube, the measurement substructure being configured to allow measurement of the at least one fluid parameter in conjunction with the at least one sensor element. Accordingly, the measurement substructure may be required for measuring the at least one fluid parameter without forming part of the at least one sensor element. In some embodiments, the at least one sensor element is comprised in the housing of the signal processing device and configured to interact with the measurement substructure. Accordingly, the measurement substructure may be exposed to the flow path and enable measurement of the at least one fluid parameter, while the at least one sensor element is not exposed to the flow path and determine the at least one fluid parameter by way of interaction with the measurement substructure.

In embodiments, the measurement substructure comprises one or more electrically conducting elements, and the at least one sensor element comprises, or is in electrical communication with, a voltmeter and/or ampere meter for measurements of fluid parameters being electrical potentials or currents. Such measurements may enable derivation of information regarding fluid flow and/or fluid charge (ion charge and concentration).

In embodiments, the measurement substructure comprises a flow restrictor in the conduit and at least one pressure communication path, and the at least one sensor element comprises a pressure sensor configured to measure a pressure in the at least pressure communication path. Accordingly, the flow restrictor exposed to urine may be conveniently disposed of, while the pressure sensor possibly guarded against contact with urine may be reused.

In embodiments, the measurement substructure comprises a window being defined by a high optical transparency relative to the surrounding catheter tube for interaction with at least one sensor element in the form of a light emitter and/or optical sensor.

In embodiments, the measurement substructure comprises an optically reflecting element, and the at least one sensor element comprises a light emitter and optical sensor for determining at least one fluid parameter in the form of an optical spectrum.

In embodiments, the measurement substructure comprises an assay with at least one predefined molecular probe, the at least one molecular probe being predefined on the basis of its binding properties and/or chemical reactivity with one or more substances or biomolecules possibly found in urine. In some embodiments, the assay emits light with a characteristic spectrum in response to binding to and/or chemical reaction with the at least one predefined molecular probe. In some embodiments, binding to and/or chemical reaction with the at least one predefined molecular probe causes a characteristic change in a recorded optical spectrum. As examples, the one or more substances or bio-molecules detected for may be Nitrite or Leucocytes.

In embodiments, the measurement substructure comprises a resilient member configured to deflect as a function of the fluid flow rate in the conduit, and the at least one sensor element comprises a camera adapted to image the deflection of the resilient member.

In embodiments, the measuring system comprises:

- a first portion comprising the at least one sensor element in the housing of the signal processing device, the at least one sensor element being configured to determine the at least one fluid parameter in the conduit and/or in a space in communication with the conduit when the signal processing device is secured in relation to the intermittent urinary catheter;
- a second portion for contacting the intermittent urinary catheter and/or its conduit;
- a barrier element between the first and the second portions, so as to prevent liquid in the conduit from coming into contact with the at least one sensor element.

In such embodiments, the at least one sensor element in the housing of the signal processing device is not exposed to liquid in the conduit allowing it to be stored and reused without a particular need for cleaning the at least one sensor element.

In embodiments, the barrier element is embedded in the catheter tube allowing convenient disposal of the barrier element possibly having been in contact with urine in the conduit.

In embodiments, the barrier element comprises a liquid impermeable flexible barrier. Such a liquid impermeable flexible barrier is advantageous in that it allows a pressure to be communicated across the barrier element without liquid crossing the barrier. Accordingly, a pressure sensor may detect a pressure in the conduit without liquid communication with the conduit. The liquid impermeable flexible barrier may be made from, e.g., natural or artificial rubber, Gore-Tex (R), or polymer such as, e.g., a technical plastic (TP) or a thermoplastic elastomer.

In embodiments, the barrier element comprises a gas conduit configured to establish a gaseous buffer between the flow path and the signal processing device for effective communication of a pressure between the flow path and the at least one sensor element of the signal processing device. In one embodiment, the gaseous buffer is atmospheric air.

In embodiments, at least one of the at least one sensor element is embedded in or integrally formed with the catheter tube. In this case, the interface for connecting the signal processing device to the at least one sensor element is configured to connect the signal processing device to the catheter tube. The interface may comprise, e.g., corresponding electrically conducting elements on each of the signal processing device and the catheter tube arranged to establish electrical contact when the housing of the signal processing device is detachably secured to the intermittent urinary catheter.

By having the at least one sensor element embedded in or integrally formed with the catheter tube, increased accuracy of determination of the at least one fluid parameter may be obtained as arrangement of the at least one sensor element relative to the conduit of the catheter tube is determined at production level. In some embodiments, a first relatively simple sensor element is embedded in or integrally formed with the catheter tube, while a second relatively complicated sensor element is arranged in the housing of the signal processing device allowing the relatively simple sensor to be disposed of with the catheter tube and the relatively complicated sensor to be re-used with the signal processing device.

In embodiments, the at least one fluid parameter comprises a pressure, wherein:

- the assembly comprises a flow restrictor in the flow path or adapted for fluid communication with the flow path and forming a through-going passage configured to restrict the flow therethrough; and wherein
- the measuring system comprises at least one pressure gauge in fluid communication with the flow path.

The combination of a flow restrictor and at least one pressure gauge in fluid communication with the flow path may allow the signal processing device or other device connected to the signal processing device to determine a fluid flow in the flow path in a relatively simple manner with increased accuracy relative to other ways of determining a fluid flow rate. Further, the fluid flow rate in the flow path is particularly useful in relation to intermittent urinary catheterisation. One reason for this is that sudden changes in the fluid flow rate may indicate that tissue of, e.g., the bladder has blocked liquid from entering the conduit at the distal insertion end and that repositioning of the intermittent urinary catheter will help to continue voiding and emptying of the bladder. Such considerations generally do not apply to an indwelling or Foley catheter, which commonly comprises a balloon at the distal insertion end for retaining the indwelling or Foley catheter in the bladder of the patient, the balloon preventing tissue from blocking entry of liquid.

The fluid flow rate may be determined on the basis of at least the pressure measured by the at least one pressure sensor and a pre-determined pressure/fluid flow rate dependency of the flow restrictor. A predetermined pressure/fluid flow rate dependency of the conduit as a whole may additionally or alternatively be used for deriving the flow rate. Additionally, the at least one pressure sensor of present embodiments enables measurement of a static pressure in the conduit or a space in communication with the conduit such as, e.g., the bladder of the user, if the flow path is cut-off downstream of the pressure gauge. The flow path may be cut-off by means of, e.g., a valve in communication with the flow path or the finger of the user at an outlet of the intermittent urinary catheter assembly. The flow restrictor may be embedded in or integrally formed with the catheter tube, or it may be comprised in the signal processing device.

In embodiments:

- the through-going passage defines an inner surface having at least a third liquid coefficient of friction;
- at least part of a perimeter of the flow path upstream of the flow restrictor has a fourth liquid coefficient of friction; and wherein
- the third liquid coefficient of friction is larger than at least the fourth liquid coefficient of friction.

In present context, the terms 'upstream' and 'downstream' refer to the flow path having a direction from the distal insertion end of the intermittent urinary catheter towards the proximal outlet end of the intermittent urinary catheter. These terms apply analogously to extensions of the flow path in fluid communication with the flow path. Accordingly, upstream refers to a flow direction towards the distal insertion end of the intermittent urinary catheter and downstream refers to a flow direction away from the distal insertion end of the intermittent urinary catheter.

The different liquid coefficients of friction may be provided by different degrees of surface roughness. In present context, the term 'liquid coefficient of friction' is to be understood as a coefficient of friction defined between the given surface and liquid water (or urine). In present embodiments, the flow restrictor comprises an area of increased liquid friction causing a pressure drop across the flow restrictor. The third and fourth liquid coefficients of friction are preferably pre-defined leading to pre-defined pressure drop/fluid flow rate dependency.

In embodiments, the third liquid coefficient of friction is brought about by the inner surface of the through-going passage forming protrusions and/or ribs for simple manufacture of the flow restriction. In embodiments, the third liquid coefficient of friction is brought about by a hydrophilic adhesion layer leading to turbulence in liquid flow past the flow restriction. In one embodiment, changes of cross-sectional area along the through-going passage lead to the third liquid coefficient of friction. The cross-sectional area of the through going passage may be larger or smaller than a first maximum cross-sectional area of the conduit. If the cross-sectional area of the through going passage is larger than the first maximum cross-section area of the conduit, this enables a more well-defined drag along the through-going passage and/or a more well-defined pressure drop across the flow restriction due to a reduced Reynolds number.

In embodiments:

- the conduit defines an internal lumen having a first maximum cross-sectional area; and

- the through-going passage has a second minimum cross-sectional area being different from, such as less than, the first maximum cross-sectional area.

The different cross-sectional areas enable a pre-defined pressure/fluid flow rate dependency of the flow restrictor. In some embodiments, the second minimum cross-sectional area of the through-going passage of the flow restrictor is sized to be in the range of 5-95% of the size of the first maximum cross-sectional area of the internal lumen of the intermittent urinary catheter.

In embodiments, the second minimum cross-sectional area of the through-going passage of the flow restrictor is sized to be in the range of 17-95% of the size of the first maximum cross-sectional area of the internal lumen of the intermittent urinary catheter enabling a pressure/fluid flow rate dependency providing a relatively large pressure variation depending on the flow rate for ease of measurement.

In embodiments, the flow restrictor comprises an orifice plate or orifice tube. In this case, the flow restrictor results in a pressure drop across the orifice plate or orifice tube being relatively simple to implement. The pressure drop/fluid flow rate dependency is defined at least in part by the difference between the first and second cross-sectional areas, i.e. the larger the difference in cross-sectional areas, the larger the pressure drop will vary in response to a change in flow rate.

In present context, the term 'orifice tube' is to be understood as an orifice plate elongated in the direction parallel to the general flow-direction through the orifice. In this way, the orifice tube forms a further extending flow restrictor than an orifice plate.

In embodiments, the second minimum cross-sectional area of the through-going passage of the orifice plate or tube is sized to be in the range of 30-95% of the size of the first maximum cross-sectional area of the internal lumen of the intermittent urinary catheter. While the difference between the first and second cross-sectional areas results in a pressure drop for determining the fluid flow, it has also been found to generally decrease the fluid flow rate in the flow path leading to increased voiding time. Present embodiments provide an advantageous compromise between accuracy of measurement and amount of voiding time prolongation.

In embodiments, the second minimum cross-sectional area of the through-going passage of the orifice plate or tube is sized to be in the range of 60-90% of the size of the first maximum cross-sectional area of the internal lumen of the intermittent urinary catheter for an improved optimisation between accuracy of measurement and amount of voiding time prolongation.

In embodiments, the flow restrictor comprises a venturi. A venturi generally results in a relatively low pressure drop across the flow restrictor resulting in a relatively small increase of voiding time as compared to, e.g., an orifice plate or tube, while a fluid flow rate dependent pressure drop is measurable at the second minimum cross-sectional area of the venturi. A venturi may also be referred to as a venturi tube.

In embodiments, the flow restrictor in the form of, e.g., an orifice plate, orifice tube or venturi is a measurement substructure embedded in or integral with the catheter tube as further described above.

In embodiments, the second minimum cross-sectional area of the through-going passage of the venturi is sized to be in the range of 17-80% of the size of the first maximum cross-sectional area of the internal lumen of the intermittent urinary catheter. Present interval of difference between the first and second cross-sectional areas have proven to be an improved compromise between accuracy of measurement and increase in voiding for a venturi flow restrictor.

In embodiments, the second minimum cross-sectional area of the through-going passage of the venturi is sized to be in the range of 17-60% of the size of the first maximum cross-sectional area of the internal lumen of the intermittent urinary catheter. The embodied interval has turned out to be a further improved compromise between accuracy of measurement and increase in voiding for a venturi flow restrictor.

In embodiments, the venturi has a second liquid coefficient of friction at least at the circumference of the second minimum cross-sectional area of the through going passage of the venturi, at least part of the internal lumen of the intermittent urinary catheter has a first liquid coefficient of friction, and the second liquid coefficient of friction is smaller than the first liquid coefficient of friction. The difference in liquid coefficients of friction may be brought about by a difference in surface roughness. The relatively small second liquid coefficient of friction in the through going passage of the venturi reduces the drag and pressure drop across the venturi without compromising accuracy of measurement. Accordingly, less increase in voiding time may be achieved for the same accuracy of measurement.

In embodiments, the at least one pressure gauge comprises:

- a first pressure gauge arranged in the flow path of the conduit or adapted for fluid communication with the flow path upstream of the flow restrictor; and
- a third pressure gauge arranged in the through-going passage,

wherein the signal processing device is configured to determine a fluid flow rate on the basis of a difference between a first pressure determined by the first pressure gauge and a third pressure determined by the third pressure gauge. Present embodiments are particularly advantageous in combination with flow restrictors increasing the fluid flow in the through-going passage, such as a venturi and allow for determining the fluid flow rate for a relatively large range of fluid flow rates.

In embodiments, the at least one pressure gauge comprises:

- a first pressure gauge arranged in the flow path of the conduit or adapted for fluid communication with the flow path upstream of the flow restrictor; and
- a second pressure gauge arranged in the flow path downstream of the flow restrictor or adapted for fluid communication with the flow path downstream of the flow restrictor,

wherein the signal processing device is configured to determine a fluid flow rate on the basis of a difference between a first pressure determined by the first pressure gauge and a second pressure determined by the second pressure gauge. Present embodiments are particularly advantageous in combination with flow restrictors primarily resulting in a pressure drop across the flow restrictor such as an orifice plate or tube and allow for determining the fluid flow rate for a relatively large range of fluid flow rates.

In embodiments, at least one fluid parameter comprises fluid flow rate, and the signal processing device is configured to determine a static pressure in the conduit or in a space in fluid communication with the conduit on the basis of the flow rate allowing simultaneous determination of the fluid flow rate and the static pressure. Accordingly, an initial static pressure in, e.g., the bladder of the user may be determined on the basis of an initial flow rate and does not require cutting off the flow path.

In embodiments, the measuring system is configured to continuously determine a loss of pressure in the fluid flow along a predetermined length of the conduit and to determine a fluid flow rate on the basis of the loss of pressure for convenient determination of the fluid flow rate.

In embodiments, the measuring system is configured to continuously determine a fluid flow rate of fluid through the conduit and to integrate the flow rate over time to obtain a measure of a total volume of urine voided from the bladder. The total volume of voided urine is particularly useful to know for a user of an intermittent urinary catheter and provides an indication whether or not further voiding is needed. The total volume of voided urine may also be an indicator of the health of the user.

In embodiments, the at least one fluid parameter comprises a pressure, and wherein at least the conduit of the intermittent urinary catheter has a pre-defined characteristic pressure drop, the measuring device comprises at least one pressure gauge in fluid communication with the flow path, and wherein the signal processing device is configured to determine a fluid flow rate on the basis of at least the pre-defined characteristic pressure drop and a pressure determined by the at least one pressure gauge. The pre-defined characteristic pressure drop is utilised to determine the characteristic pressure drop/fluid flow rate dependency and allows the fluid flow rate to be determined. The pre-defined characteristic pressure drop may be pre-determined on the basis of a reference measurement on the embodied intermittent urinary catheter, or the embodied intermittent urinary catheter may be a standardised intermittent urinary catheter wherein the pre-defined characteristic pressure drop is based on a reference measurement on another standardised intermittent urinary catheter.

In embodiments, the measuring device comprises a valve configured to allow a variable cross-sectional area of fluid flow in fluid communication with the flow path. This allows the user to vary the fluid flow rate in the flow path by adjusting the valve. In one embodiment, the valve is variable between a set of different pre-determined cross-sectional areas. In this embodiment, the valve may serve as a flow restrictor in combination with a pressure gauge. In this case, the flow rate may be determined on the basis of at least the pre-determined cross-sectional area of the valve and a pressure determined by the pressure gauge. Accordingly, if more accuracy of measurement is desired, the user may reduce the pre-determined cross-sectional area, and if a larger fluid flow rate is desired the user may increase the predetermined cross-sectional area.

In embodiments, the valve is configured to automatically vary the cross-sectional area for the fluid flow on the basis of a determined fluid flow rate or pressure in the flow path. In this case, the valve may serve as a flow restrictor and the valve can automatically increase the cross-sectional area to increase the fluid flow rate in response to a measured fluid flow rate or pressure below a first threshold value, and automatically decrease the cross-sectional area to increase accuracy of measurement in response to a measured fluid flow rate or pressure above a second threshold value. This allows automatic optimisation of the relationship between accuracy of measurement and voiding time.

In a third aspect, embodiments provide a catheter system comprising a disposable intermittent urinary catheter according to any embodiment of the second aspect and a package for accommodating the intermittent urinary catheter therein in a sterile condition alleviating the need for sterilising the intermittent urinary catheter after removal from the package.

In embodiments, at least the insertable portion of the catheter tube comprises a hydrophilic surface coating, wherein the package accommodates an amount of a liquid swelling medium. Accordingly, the liquid swelling medium is able to activate the hydrophilic surface coating to achieve a very lubricious surface that has very low-friction when the catheter is to be inserted already in the package. In embodiments, the package comprises a gas-impermeable barrier enclosing an inner cavity accommodating the intermittent urinary catheter for improved shelf-life of the catheter system. In one embodiment, the liquid swelling medium is a water-based substance, such as sterile water, saline-solution, or any water-based liquid.

In a fourth aspect, non-claimed embodiments here below provide an intermittent urinary catheterisation assembly comprising:

- an intermittent urinary catheter comprising:

    ◦ a catheter tube comprising an insertable portion intended for insertion into a user's urethra and a non-insertable portion not intended for insertion into the user's urethra;
    ◦ a conduit extending longitudinally within the catheter tube and defining at least part of a flow path from a distal insertion end of the intermittent urinary catheter to a proximal outlet end thereof;

- a measuring system securely connected to or integrated with the non-insertable portion of the catheter tube for determining at least one fluid parameter in the flow path, comprising

    ◦ at least one sensor element configured to determine the at least one fluid parameter in the conduit and/or in a space in communication with the conduit;
    ◦ a signal processing device comprising a data communication interface.

Thanks to the provision of the at least one sensor element and the signal processing device a user of an embodied intermittent urinary catheter assembly is enabled to determine at least one fluid parameter in the conduit of the intermittent urinary catheter and/or in a space in communication with the conduit. The at least one fluid parameter may be relevant for several aspects of intermittent catheterisation and provide information on, e.g., the flow and/or amount of urine expelled during intermittent catheterisation, the filing degree of the bladder prior to intermittent catheterisation, the hydration level of the user, the presence and/or concentration of one or more analytes. Accordingly, the user is provided with improved utility of the intermittent urinary catheter assembly and may be able to make informed choices based on the information determined by the at least one sensor element and signal processing device. Further, thanks to the provision of the measuring system being securely connected to or integrated with the non-insertable portion of the catheter tube a user is able to determine the at least one fluid parameter without establishment of a connection between the catheter tube and measuring system.

At least one of the at least one sensor element may be imbedded in or integral with the catheter tube. Alternatively or additionally at least one of the at least one sensor element may be comprised in a housing of the signal processing device.

In embodiments, the at least one fluid parameter comprises at least one of: pressure, fluid flow rate, particle size, particle count, particle distribution, particle concentration, particle characteristics, analyte concentration, refractive index, conductivity, temperature, absorption spectrum, refraction spectrum, viscosity, pH. Each parameter may be used to determine information relevant to the user. A sensed pressure and/or fluid flow rate may be used to determine, e.g., a urine fluid flow rate, a pressure in the bladder, an initial pressure in the bladder prior to urine evacuation, a measure of the degree of expansion of the bladder, the amount of urine evacuated during intermittent catheterisation, and/or if urine evacuation has suddenly stopped. The different particle characteristics allow derivation of information regarding the condition of the user. For instance, certain particle sizes/counts/distributions/concentrations/characteristics may be indicators of a condition or disease. For example, a relatively high amount of protein in the urine could be signs of damage to the glomeruli. Fluid parameters such as analyte concentration, absorption spectra (colour) and conductivity may be used to determine a hydration level of the user, which in turn may help the user to prevent or alleviate dehydration, which helps to prevent infection of the bladder and/or urinary tract. The particle characteristics and spectra may also be used to establish presence and concentration of cells in the urine such as leukocytes. Any more than trace amounts of leukocytes in the urine may indicate a urinary tract or bladder infection. Such conditions are particularly relevant to detect for users of intermittent urinary catheters as they are generally relatively exposed to such infections due to the repeated introduction of an intermittent urinary catheter in the urethra.

In embodiments, the signal processing device comprises wired or wireless connection means for connecting to- and transmitting data to another electronic device such as, e.g., a portable electronic device such as a smartphone or laptop, or a stationary computer or server for further data analysis and/or output.

In embodiments, the measuring system comprises data output means. The data output means may comprise a visual output unit possibly with resolution of colour. In embodiments, the data output means comprises tactile feedback means. The tactile feedback means may particularly comprise a vibrator. The tactile feedback is able to provide feedback to the user without requiring visual contact with the intermittent urinary catheterisation assembly. Such feedback is particularly advantageous in relation to intermittent catheterisation as it is commonly done by the user him- or herself and visual inspection of the intermittent catheterisation assembly may be difficult and/or inconvenient. In one embodiment, the measuring system is configured to provide tactile feedback to the user in response to detection of a fluid flow rate, and/or a derivative thereof, below a first threshold in the conduit, and/or provide tactile feedback in response to detection of a fluid flow rate, or a derivative thereof, above a second threshold in the conduit for increased user convenience.

In embodiments, the intermittent urinary catheter assembly further comprises a package assembly for accommodating the intermittent urinary catheter and the signal processing device, wherein the signal processing device is operatively connected to or operatively integrated with the intermittent urinary catheter in the package assembly when the package assembly is in a closed state. In this case, the intermittent urinary catheter assembly will be able to determine the at least one fluid parameter without requiring establishment of operational connection between the intermittent urinary catheter and the signal processing device after opening of the package. In one embodiment, the intermittent urinary catheter is stored in the package assembly in a ready-to-use condition allowing insertion of the intermittent urinary catheter and determination of the at least one fluid parameter directly upon removal of the assembly from the package requiring less handling prior to insertion.

In embodiments, the intermittent urinary catheter is configured to rest in the user's urethra for a period of time not exceeding 15 minutes. This allows the intermittent urinary catheter to comprise, e.g., coatings that do not allow extended duration of catheterisation required for indwelling catheterisation.

In embodiments, at least an outer surface of the insertable portion of the catheter tube comprises a hydrophilic surface coating. Such hydrophilic coating allows particularly convenient intermittent urinary catheterisation. In embodiments, the package assembly comprises an amount of liquid swelling medium for activation of the hydrophilic surface to provide the intermittent urinary catheter assembly in a ready-to-use state when the package assembly is in a closed state.

The hydrophilic coating may be provided only on the insertable part of the catheter. The hydrophilic surface coating is of the kind which, when hydrated or swelled using a swelling medium, reduces the friction on the surface area of the catheter which is intended to be inserted into the urinary channel of a user corresponding to the insertable part of the catheter.

An intermittent hydrophilic catheter differs from an indwelling catheter in that the hydrophilic surface coating of such a catheter is not suitable for indwelling use, because the surface coating tends to stick inside the mucosa of the urethra if left inside the body for a period exceeding 5-20 minutes, due to the hydrophilic coating transforming from being highly lubricious when fully wetted (95% weight water) to being adhesive when the hydration level of the coating is reduced (<75% weight water).

In some embodiments of the fourth aspect, at least an outer surface of the insertable portion of the catheter tube comprises a hydrophilic surface coating, and the urinary catheter assembly comprises a package assembly comprising an outer package for accommodating the intermittent urinary catheter and the signal processing device, an amount of liquid swelling medium, and a gas- and/or liquid impermeable inner barrier, wherein the inner barrier in a closed state of the outer package is arranged to prevent the liquid swelling medium from reaching the signal processing device. Accordingly, the hydrophilic coating of the intermittent urinary catheter assembly is allowed to be provided in a ready-to-use state without exposing the signal processing device to the liquid swelling medium when the package assembly is in a closed state.

In embodiments, the catheter tube is a single-lumen tube, of which the conduit constitutes the sole passage between the distal insertion end and the proximal outlet end of the intermittent urinary catheter. Such single-lumen tube catheters are generally not applicable for indwelling use because of the lack of a passage for conducting air to the balloon of an indwelling or Foley urinary catheter.

In embodiments, the measuring system comprises a measurement substructure embedded in or integral with the catheter tube, the measurement substructure being configured to allow measurement of the at least one fluid parameter in conjunction with the at least one sensor element. Accordingly, the measurement substructure may be required for measuring the at least one fluid parameter without forming part of the at least one sensor element. In some embodiments, the at least one sensor element is comprised in the signal processing device and configured to interact with the measurement substructure. Accordingly, the measurement substructure may be exposed to the flow path and enable measurement of the at least one fluid parameter, while the at least one sensor element is not exposed to the flow path and determine the at least one fluid parameter by way of interaction with the measurement substructure.

In embodiments, the measurement substructure comprises one or more electrically conducting elements, and the at least one sensor element comprises, or is in electrical communication with, a voltmeter and/or ampere meter for measurements of fluid parameters being electrical potentials or currents. Such measurements may enable derivation of information regarding fluid flow, fluid charge (ion charge and concentration).

In embodiments, the measurement substructure comprises a flow restrictor in the conduit and at least one pressure communication path, and the at least one sensor element comprises a pressure sensor configured to measure a pressure in the at least one pressure communication path.

In embodiments, the measurement substructure comprises a window being defined by a high optical transparency relative to the surrounding catheter tube for interaction with at least one sensor element in the form of a light emitter and/or optical sensor.

In embodiments, the measurement substructure comprises an optically reflecting element, and the at least one sensor element comprises a light emitter and optical sensor for determining at least one fluid parameter in the form of an optical spectrum.

In embodiments, the measurement substructure comprises an assay with at least one predefined molecular probe, the at least one molecular probe being predefined on the basis of its binding properties and/or chemical reactivity with one or more substances or biomolecules possibly found in urine. In some embodiments, the assay emits light with a characteristic spectrum in response to binding to and/or chemical reaction with the at least one predefined molecular probe. In some embodiments, binding to and/or chemical reaction with the at least one predefined molecular probe causes a characteristic change in a recorded optical spectrum. As examples, the one or more substances or bio-molecules detected for may be Nitrite or Leucocytes.

In embodiments, the measurement substructure comprises a resilient member configured to deflect as a function of the fluid flow rate in the conduit, and the at least one sensor element comprises a camera adapted to image the deflection of the resilient member.

In embodiments, at least one of the at least one sensor element is embedded in or integrally formed with the catheter tube. By having the at least one sensor element imbedded in or integrally formed with the catheter tube, increased accuracy of determination of the at least one fluid parameter may be obtained as arrangement of the at least one sensor element relative to the conduit of the catheter tube is determined at production level.

In embodiments, the at least one fluid parameter comprises a pressure, and:

- the assembly comprises a flow restrictor in the flow path or adapted for fluid communication with the flow path and forming a through-going passage configured to restrict the flow therethrough; and
- the measuring system comprises at least one pressure gauge in fluid communication with the flow path.

The combination of a flow restrictor and at least one pressure gauge in fluid communication with the flow path may allow the signal processing device or other device connected to the signal processing device to determine a fluid flow in the flow path in a relatively simple manner with increased accuracy relative to other ways of determining a fluid flow rate. Further, the fluid flow rate in the flow path is particularly useful in relation to intermittent urinary catheterisation. One reason for this is that sudden changes in the fluid flow rate may indicate that tissue of, e.g., the bladder has blocked liquid from entering the conduit at the distal insertion end and that repositioning of the intermittent urinary catheter will help to continue voiding and emptying of the bladder. Such considerations generally do not apply to an indwelling or Foley catheter, which commonly comprises a balloon at the distal insertion end for retaining the indwelling or Foley catheter in the bladder of the patient, the balloon preventing tissue from blocking entry of liquid.

The fluid flow rate may be determined on the basis of at least the pressure measured by the at least one pressure sensor and a pre-determined pressure/-fluid flow rate dependency of the flow restrictor. A pre-determined pressure/fluid flow rate dependency of the conduit as a whole may additionally or alternatively be used for deriving the flow rate. Additionally, the at least one pressure sensor of present embodiments enables measurement of a static pressure in the conduit or a space in communication with the conduit such as, e.g., the bladder of the user, if the flow path is cut-off downstream of the pressure gauge. The flow path may be cut-off by means of, e.g., a valve in communication with the flow path or the finger of the user at an outlet of the intermittent urinary catheter assembly. The flow restrictor may be embedded in or integrally formed with the catheter tube, or it may be comprised in the signal processing device.

In embodiments:

- the through-going passage defines an inner surface having at least a third liquid coefficient of friction;
- at least part of a perimeter of the flow path upstream of the flow restrictor has a fourth liquid coefficient of friction; and wherein
- the third liquid coefficient of friction is larger than at least the fourth liquid coefficient of friction.

The different liquid coefficients of friction may be provided by different degrees of surface roughness. In present context, the term 'liquid coefficient of friction' is to be understood as a coefficient of friction defined between the given surface and liquid water (or urine). In present embodiments, the flow restrictor comprises an area of increased liquid friction causing a pressure drop in the flow path. The third and fourth liquid coefficients of friction are preferably pre-defined leading to pre-defined pressure drop/fluid flow rate dependency.

In embodiments, the third liquid coefficient of friction is brought about by the inner surface of the through-going passage forming protrusions and/or ribs for simple manufacture of the flow restriction. In embodiments, the third liquid coefficient of friction is brought about by a hydrophilic adhesion layer leading to turbulence in liquid flow past the flow restriction. In one embodiment, changes of cross-sectional area along the through-going passage lead to the third liquid coefficient of friction. The cross-sectional area of the through going passage may be larger or smaller than a first maximum cross-sectional area of the conduit. If the cross-sectional area of the through going passage is larger than the first maximum cross-section area of the conduit, this enable a more well-defined drag along the through-going passage and/or a more well-defined pressure drop across the flow restriction due to a reduced Reynolds number.

In embodiments:

- the conduit defines an internal lumen having a first maximum cross-sectional area; and wherein

- the through-going passage has a second minimum cross-sectional area.

The different cross-sectional areas enable a pre-defined pressure/fluid flow rate dependency of the flow restrictor. In some embodiments, the second minimum cross-sectional area of the through-going passage of the flow restrictor is sized to be in the range of 5-95% of the size of the first maximum cross-sectional area of the internal lumen of the intermittent urinary catheter.

In embodiments, the second minimum cross-sectional area of the through-going passage of the flow restrictor is sized to be in the range of 17-95% of the size of the first maximum cross-sectional area of the internal lumen of the intermittent urinary catheter enabling a pressure/fluid flow rate dependency providing a relative large pressure variation depending on the flow rate for ease of measurement.

In embodiments, wherein the flow restrictor comprises an orifice plate or orifice tube. In this case, the flow restrictor results in a pressure drop across the orifice plate or orifice tube being relatively simple to implement. The pressure drop/fluid flow rate dependency is defined at least in part by the difference between the first and second cross-sectional areas, i.e. the larger the difference in cross-sectional areas, the larger the pressure drop will vary in response to a change in flow rate.

In embodiments, the second minimum cross-sectional area of the through-going passage of the orifice plate or tube is sized to be in the range of 30-95% of the size of the first maximum cross-sectional area of the internal lumen of the intermittent urinary catheter. While the difference between the first and second cross-sectional areas results in a pressure drop for determining the fluid flow, it has also been found to generally decrease the fluid flow rate in the flow path leading to increased voiding time. Present embodiments provide an advantageous compromise between accuracy of measurement and amount of voiding time prolongation.

In embodiments, the second minimum cross-sectional area of the through-going passage of the orifice plate or tube is sized to be in the range of 60-90% of the size of the first maximum cross-sectional area of the internal lumen of the intermittent urinary catheter for an improved optimisation between accuracy of measurement and amount of voiding time prolongation.

In embodiments, the flow restrictor comprises a venturi. A venturi generally results in a relatively low pressure drop across the flow restrictor resulting in a relatively small increase of voiding time as compared to, e.g., an orifice plate or tube, while a fluid flow rate dependent pressure drop is measurable at the second minimum cross-sectional area of the venturi. A venturi may also be referred to as a venturi tube.

In embodiments, the flow restrictor in the form of, e.g., an orifice plate, orifice tube or venturi is a measurement substructure embedded in or integral with the catheter tube as further described above.

In embodiments, the second minimum cross-sectional area of the through-going passage of the venturi is sized to be in the range of 17-80% of the size of the first maximum cross-sectional area of the internal lumen of the intermittent urinary catheter. Present interval of difference between the first and second cross-sectional areas have proven to be an improved compromise between accuracy of measurement and increase in voiding for a venturi flow restrictor.

In embodiments, the second minimum cross-sectional area of the through-going passage of the venturi is sized to be in the range of 30-60% of the size of the first maximum cross-sectional area of the internal lumen of the intermittent urinary catheter for an even better compromise between accuracy of measurement and increase in voiding for a venturi flow restrictor.

In embodiments, the venturi has a second liquid coefficient of friction at least at the circumference of the second minimum cross-sectional area of the through going passage of the venturi, wherein at least part of the internal lumen of the intermittent urinary catheter has a first liquid coefficient of friction, and wherein the second liquid coefficient of friction is smaller than the first liquid coefficient of friction. The difference in liquid coefficients of friction may be brought about by a difference in surface roughness. The relatively small second liquid coefficient of friction in the through going passage of the venturi reduces the drag and pressure drop across the venturi without compromising accuracy of measurement. Accordingly, less increase in voiding time may be achieved for the same accuracy of measurement.

In embodiments, the at least one pressure gauge comprises:

- a first pressure gauge arranged in the flow path of the conduit or adapted for fluid communication with the flow path upstream of the flow restrictor; and
- a third pressure gauge arranged in the through-going passage,

and wherein the signal processing device is configured to determine a fluid flow rate on the basis of a difference between a first pressure determined by the first pressure gauge and a third pressure determined by the third pressure gauge. Present embodiments are particularly advantageous in combination with flow restrictors increasing the fluid flow rate at in the through-going passage, such as a venturi and allow for determining the fluid flow rate for a relatively large range of fluid flow rates.

In embodiments, the at least one pressure gauge comprises:

- a first pressure gauge arranged in the flow path of the conduit or adapted for fluid communication with the flow path upstream of the flow restrictor; and
- a second pressure gauge arranged in the flow path downstream of the flow restrictor or adapted for fluid communication with the flow path downstream of the flow restrictor,

and wherein the signal processing device is configured to determine a fluid flow rate on the basis of a difference between a first pressure determined by the first pressure gauge and a second pressure determined by the second pressure gauge. Present embodiments are particularly advantageous in combination with flow restrictors primarily resulting in a pressure drop across the flow restrictor such as an orifice plate or tube, allow for determining the fluid flow rate for a relatively large range of fluid flow rates.

In embodiments, the measuring system is configured to continuously determine a loss of pressure in the fluid flow along a predetermined length of the conduit and to determine a fluid flow rate on the basis of the loss of pressure, which allows determination of the fluid flow rate without the necessity of a dedicated flow restrictor.

In embodiments, the measuring system is configured to continuously determine a fluid flow rate of fluid through the conduit and to integrate the flow rate over time to obtain a measure of a total volume of urine voided from the bladder. The total volume of voided urine is particularly useful to know for a user of an intermittent urinary catheter and provides an indication whether or not further voiding is needed. The total volume of voided urine may also be an indicator of the health of the user.

In embodiments, the at least one fluid parameter comprises a pressure, wherein at least the conduit of the intermittent urinary catheter has a pre-defined characteristic pressure drop, the measuring device comprises at least one pressure gauge in fluid communication with the flow path, and wherein the signal processing device is configured to determine a fluid flow rate on the basis of at least the pre-defined characteristic pressure drop and a pressure determined by the at least one pressure gauge. The pre-defined characteristic pressure drop is utilised to determine the characteristic pressure drop/fluid flow rate dependency and allows the fluid flow rate to be determined. The pre-defined characteristic pressure drop may be pre-determined on the basis of a reference measurement on the embodied intermittent urinary catheter, or the embodied intermittent urinary catheter may be a standardised intermittent urinary catheter wherein the pre-defined characteristic pressure drop is based on a reference measurement on another standardised intermittent urinary catheter.

In embodiments, the measuring device comprises a valve configured to allow a variable cross-sectional area of fluid flow in fluid communication with the flow path. This allows the user to vary the fluid flow rate in the flow path by adjusting the valve. In one embodiment, the valve is variable between a set of different pre-determined cross-sectional areas. In this embodiment, the valve may serve as a flow restrictor in combination with a pressure gauge. In this case, the flow rate may be determined on the basis of at least the pre-determined cross-sectional area of the valve and a pressure determined by the pressure gauge. Accordingly, if more accuracy of measurement is desired, the user may reduce the pre-determined cross-sectional area, and if a larger fluid flow rate is desired the user may increase the pre-determined cross-sectional area.

In embodiments, the valve is configured to automatically vary the cross-sectional area for the fluid flow on the basis of a determined fluid flow rate or pressure in the flow path. In this case, the valve may serve as a flow restrictor and the valve can automatically increase the cross-sectional area to increase the fluid flow rate in response to a measured fluid flow rate or pressure below a first threshold value, and automatically decrease the cross-sectional area to increase accuracy of measurement in response to a measured fluid flow rate or pressure above a second threshold value. This allows automatic optimisation of the relationship between accuracy of measurement and voiding time.

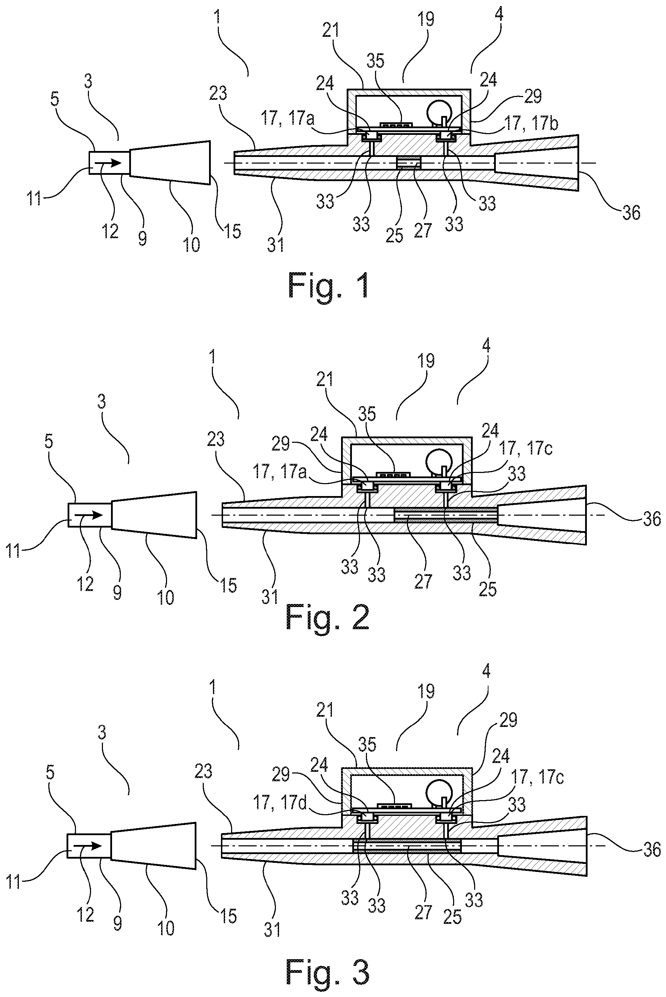

Fig. 1 illustrates a schematic cross-sectional view of an embodied intermittent urinary catheter assembly 1 with an intermittent urinary catheter 3 and a measuring system 4. The intermittent urinary catheter 3 comprises a catheter tube 5. The catheter tube 5 comprises an insertable portion 7 intended for insertion into a user's urethra, a non-insertable portion 9 not intended for insertion into the user's urethra, and a connecting portion 10 being integral with the non-insertable portion 9. The intermittent urinary catheter 3 further comprises a conduit 11 extending longitudinally within the catheter tube 5. The conduit 11 defines a flow path 12 from a distal insertion end 13 of the catheter 3 to a proximal outlet end 15 thereof. Only the non-insertable portion 9 of the catheter tube 5 and the connecting portion 10 and the proximal outlet end 15 of the intermittent urinary catheter 3 are illustrated, while the insertable portion 7 and the distal insertion end 13 of the catheter tube 5 extend outside the area illustrated in Fig. 1.

The intermittent urinary catheter assembly 1 of Fig. 1 further comprises a measuring system 4 for determining at least one fluid parameter in the flow path 12. In the illustrated embodiment, the measuring system 4 comprises two sensor elements 17 configured to determine the at least one fluid parameter in the conduit 11 and/or in a space in communication with the conduit 11. The measuring system 4 further comprises signal processing device 19 with a housing 21. The housing 21 has an engagement mechanism 23 that is able to detachably secure the signal processing device 19 in relation to the connecting portion 10 of the intermittent urinary catheter 3. The signal processing device 19 further comprises an interface 24 for connecting the signal processing device 19 to the sensor elements 17. In the embodiment of Fig. 1, the two sensor elements 17 are comprised within the housing 21, in which case the interface 24 connects the sensor elements 17 to the signal processing device 19 within the housing 21. In the embodiment of Fig. 1, the engagement mechanism 23 is detachably secured to the connecting portion 10 by firmly inserting the protruding engagement mechanism 23 in a receiving cavity of the connecting portion 10.

In one embodiment, the intermittent urinary catheter 3 is configured to rest in the user's urethra for a period of time not exceeding 15 minutes and at least an outer surface of the insertable portion 7 of the catheter tube 5 comprises a hydrophilic surface coating. The illustrated catheter tube 5 is a single-lumen tube, of which the conduit constitutes the sole passage between the distal insertion end 13 and the proximal outlet end 15.

In the embodiment of Fig. 1, the intermittent urinary catheter assembly 1 comprises a flow restrictor 25 in the signal processing device 19. The flow restrictor 25 is in fluid communication with the flow path 12 when the signal processing device 19 is detachably secured in relation to the catheter tube 5. The conduit 11 comprises an internal lumen having a first maximum cross-sectional area and the flow restrictor 25 forms a through-going passage 27 having a second minimum cross-sectional area. The flow restrictor 25 of Fig. 1 may be referred to as an orifice tube. In one embodiment, the second minimum cross-sectional area of the through-going passage 27 of the orifice tube is sized to be in the range of 17-95% of the size of the first maximum cross-sectional area of the internal lumen of the intermittent urinary catheter 3, in one embodiment 30-95%, and in one embodiment 60-90%.

In Fig. 1, the first sensor element 17a is a first pressure gauge 17a adapted for fluid communication with the flow path 12 upstream of the flow restrictor 25, and the second sensor element 17b is a second pressure gauge 17b adapted for fluid communication with the flow path 12 downstream of the flow restrictor 25. When in use, the signal processing device 19 determines a flow rate on the basis of a difference between a first pressure determined by the first pressure gauge 17a and a second pressure determined by the second pressure gauge 17b.

Generally, in determining the fluid flow rate, the signal processing device 19 determines the flow rate using the flow characteristics of the orifice tube, which may be derived using Bernoulli's principle to yield:

$$Q = K1\sqrt{(\Delta P)}$$

in which K1 is a specific constant measured for urine, the dimensions of the flow path and the extension thereof including the first maximum cross-sectional area of the internal lumen and the second minimum cross-sectional area of the trough-going passage 27. $\Delta P$ is the difference between the first and second pressures, and Q is the fluid flow rate. The signal processing device 19 may have a set of pre-determined values for K1, each pre-determined value for K1 corresponding to a specific type and size of intermittent urinary catheter 3. The signal processing device 19 is in some embodiments configured to recognise the type and size of intermittent urinary catheter 3 and automatically select the correct pre-determined value for K1, and in some embodiments the signal processing device 19 comprises means for receiving user input regarding the type and size of intermittent urinary catheter 3.

In the embodiment of Fig. 1, the measuring system 4 comprises a first portion 29 with the sensor elements 17 in the housing 21 and a second portion 31 for contacting the conduit 11. A barrier element 33 prevents liquid in the conduit 11 from coming into contact with the sensor elements 17. In Fig. 1, the barrier element 33 comprises a flexible membrane and an amount of sealed atmospheric air.

In embodiments, the signal processing device 19 comprises a data processing unit 35 and a power source, such as a battery or photovoltaic system.

Fig. 2 illustrates a schematic cross-sectional view of an embodied intermittent urinary catheter assembly 1 being a variant of that in Fig. 1. In Fig. 2, the flow restrictor 25 is arranged differently relative to the sensor elements 17. In the embodiment of Fig. 2, the first pressure gauge 17a is adapted for fluid communication with the flow path 12 upstream of the flow restrictor 25, while a third pressure gauge 17c is arranged in the through-going passage 27 of the flow restrictor 25. The relatively small second minimum cross-sectional area of the through-going passage 27 works to increase the fluid velocity therethrough, which in turn results in a pressure drop from the first 17a to the third pressure gauge 17c. In practice, a pressure from the first 17a to third pressure gauge 17c also occurs due to the sudden change in cross-sectional area and possibly also surface roughness. Accordingly, an increased pressure difference per change in flow rate may be attained in the embodiment of Fig. 2 leading to greater accuracy of measurement. The relative arrangement of the sensor elements 17 and the flow restrictor 25 illustrated in Fig. 2 is not limited to a particular embodiment but may generally be applied in different embodiments and aspects.

Fig. 3 illustrates a schematic cross-sectional view of an embodied intermittent urinary catheter assembly 1 being a variant of that in Figs. 1 and 2, wherein the flow restrictor 25 is arranged differently relative to the sensor elements 17. In the embodiment of Fig. 3 a third pressure gauge 17c and fourth pressure gauge 17d are arranged in the through-going passage 27 of the flow restrictor 25 having the second minimum cross-sectional area.

Further, the inner surface of the through-going passage 27 has a third liquid coefficient of friction larger than a fourth liquid coefficient of friction upstream of the flow restrictor 25. The flow restrictor 25 creates a pressure drop due to relatively large third coefficient of friction. In this case, the flow rate Q can be determined by the signal processing device 25 using

$$Q = K2\Delta P$$

where K2 is another specific constant depending the third and fourth coefficients of friction as well as other types of flow loss in the flow path and the extension thereof. Again, the signal processing device 19 may have a set of pre-determined values for K2, each pre-determined value for K2 corresponding to a specific type and size of intermittent urinary catheter 3. The signal processing device 19 is in some embodiments configured to recognise the type and size of intermittent urinary catheter 3 and automatically select the correct pre-determined value for K2, and in some embodiments the signal processing device 19 comprises means for receiving user input regarding the type and size of intermittent urinary catheter 3.

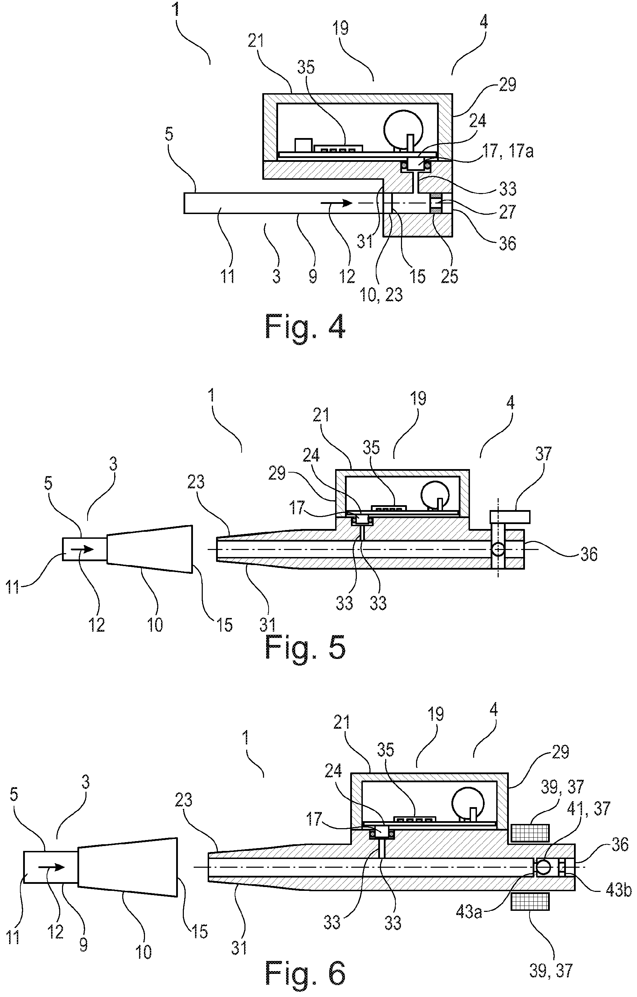

Fig. 4 illustrates a schematic cross-sectional view of an embodied intermittent urinary catheter assembly 1. In the embodiment of Fig. 4, the at least one sensor element 17 of the measuring system 4 is a first pressure gauge 17a adapted for fluid communication with the flow path 12 and conduit 11 upstream of the flow restrictor 25. The flow restrictor 25 defines a through-going passage 27 having a second minimum cross-sectional area and is arranged in the vicinity of a signal processing device outlet 36. In embodiments, the distance between the flow restrictor 25 and the signal processing device outlet 36 is less than the diameter of the flow path between the flow restrictor 25 and the outlet 36. The pressure downstream of the flow restrictor 25 will in this case be equal to or very close to ambient pressure and the pressure drop across the flow restrictor 25 is determined on the basis of the difference between the first pressure measured by the first pressure gauge 17a and the ambient pressure. The ambient pressure may be measured or set to a predefined value. In one embodiment, the first pressure gauge 17a measures the first pressure relative to the ambient pressure in which case the fluid flow rate is determined on the basis of the first pressure.

In the illustrated example in Fig. 4, the engagement mechanism 23 is detachably secured to the connecting portion 10 by firmly inserting the protruding connecting portion 10 of the intermittent urinary catheter 3 in a receiving part of the engagement mechanism 23.

Fig. 5 illustrates a schematic cross-sectional view of an embodied intermittent urinary catheter assembly 1. In the embodiment of Fig. 5, the measuring system 4 comprises a valve 37 in fluid communication with the flow path 12. The valve 37 allows a variable cross-sectional area of fluid flow in fluid communication with the flow path 12 by gradually turning the valve 37 in a plane perpendicular to the plane of the illustration. When the connecting portion 10 is detachably secured to the engagement mechanism 23 and the valve 37 is set to completely shut off flow in the flow path 12 and conduit 11, the pressure measured by the pressure gauge 17, a static pressure in the flow path 12 is determined on the basis of the measured pressure. In one embodiment, a static pressure in a space in communication with the flow path 12 and conduit 11 is determined on the basis of the measured pressure. In all embodiments, the space in communication with the flow path 12 and conduit 11 can be the bladder of the user, when the intermittent urinary catheter 3 is inserted in the urethra of the user. In one embodiment, the signal processing device 19 comprises an air outlet not allowing liquid passage due to a pore size, wherein the air outlet is arranged to allow liquid to reach the pressure gauge 17 while the valve 37 is closed.

In one embodiment, the valve 37 is variable between a set of different pre-determined cross-sectional areas by turning the valve 37 between a set of pre-determined degrees of rotation, which may be marked, e.g., by visual indicators on the valve 37 or clicks. In this embodiment, the valve 37 also serves as a flow restrictor 37 in combination with the pressure gauge 17. The fluid flow rate is then determined on the basis of at least the pre-determined cross-sectional area of the valve 37 and a pressure measured by the pressure gauge 17. In one embodiment, the signal processing device 19 has a user interface (not shown) that allows the user to input, e.g., the degree of rotation of the valve 37 for determination of the fluid flow rate.

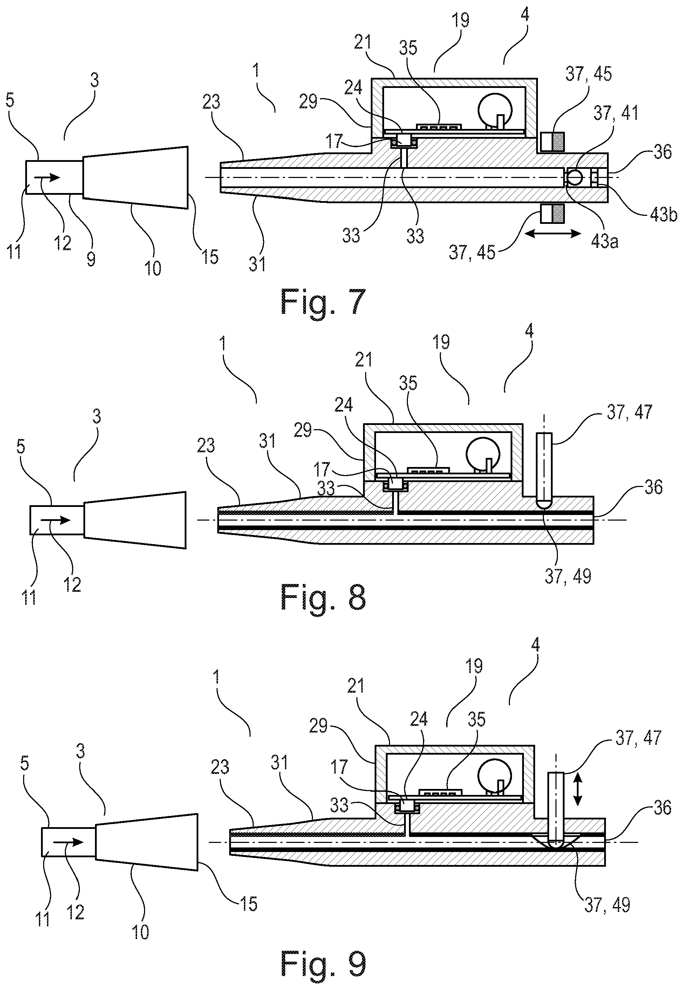

Figs. 6-9 illustrate embodiments with different types of valves 37. In the embodiment of Fig. 6, the valve 37 is of an electronic type. In one embodiment, a coil 39 is wrapped around part of the signal processing device 19 to interact with a ferromagnetic valve element 41, e.g. a ball, arranged between first and second immobilizing elements 43a, 43b. By controlling the current in the coil, the ferromagnetic valve element 41 can be pushed towards the first immobilizing element 43a in which case the valve 37 shuts off liquid flow, or it can be shifted towards (or allowed to be pushed towards-) the second immobilizing element 43b which allows liquid to flow past the valve 37. In Fig. 7, the coil 39 of the embodiment of Fig. 6 has been replaced by a ferromagnetic manually slide-able element 45 allowing a user to selective push the ferromagnetic valve element 41 towards the first or second immobilizing elements 43a, 43b. In Figs. 8 and 9 the valve 37 is in the form of a pushing rod 47 in combination with a flexible valve membrane 49 allowing the valve 37 to be opened with the flexible valve membrane 49 not restricting the flow as illustrated in Fig. 8, and allowing the valve 37 to be closed by pushing down the pushing rod 47 to force the flexible valve membrane 49 to shut off liquid flow past the valve 37 as illustrated in Fig. 9.

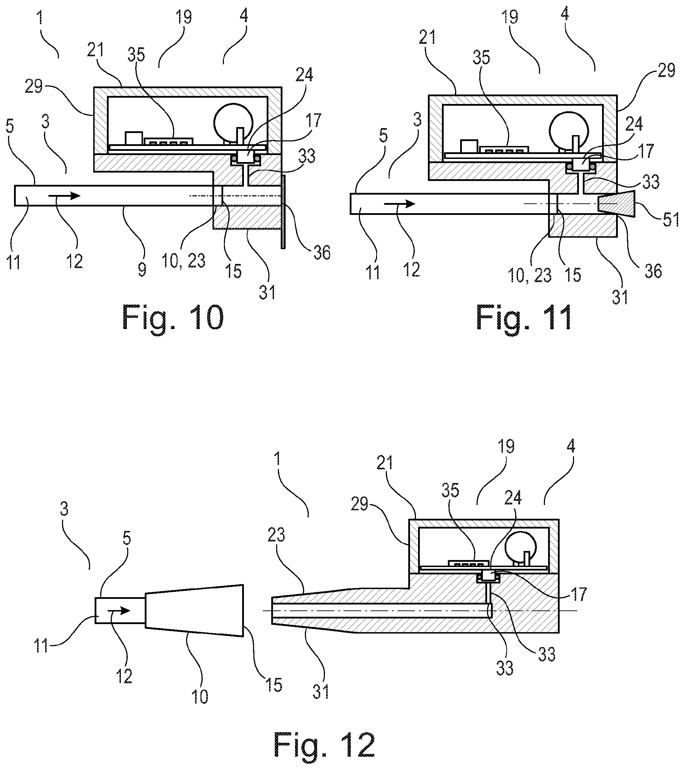

Figs. 10 and 11 illustrate embodiments of an intermittent urinary catheter assembly 1, wherein the measuring system 4 comprises a pressure gauge 17 in fluid communication with the flow path 12 and conduit 11. When the connecting portion 10 is detachably secured to the engagement mechanism 23, the finger of the user in Fig. 10 and a plug 51 in Fig. 11 is used to shut off the liquid flow at the signal processing device outlet 36, in which case the pressure measured by the pressure gauge 17, a static pressure in the flow path 12 is determined on the basis of the measured pressure. In one embodiment, a static pressure in a space in communication with the flow path 12 and conduit 11 is determined on the basis of the measured pressure. The space in communication with the path 12 and conduit 11 can be the bladder of the user, when the intermittent urinary catheter 3 is inserted in the urethra of the user.

Fig. 12 illustrates an embodiment of intermittent urinary catheter assembly 1 in which the measuring system 4 is configured to prevent flow through the signal processing device 19 comprising the at least one sensor element 17 in the form of a pressure gauge 17. In this case, when the intermittent urinary catheter 3 is detachably secured to the signal processing device 19 liquid flow in the flow path 12 and conduit 11 is hindered. Accordingly, the pressure measured by the pressure gauge 17, a static pressure in the flow path 12 is determined on the basis of the measured pressure.

Figs. 1-12 have been illustrated and described in relation to the first aspect. Variations of the illustrated embodiments, wherein the measuring system 4 is securely connected to or integrated with the non-insertable portion 9 of the catheter tube 5 are also envisioned as in embodiments of the fourth aspect. In these variations the connecting portion 10 and the engagement mechanism 23 are exchanged with a permanent secure connection where the connecting portion 10 and the engagement mechanism 23 would detachably connect, or the catheter tube 5 and the signal processing device 19 are integrally formed where the connecting portion 10 and the engagement mechanism 23 would detachably connect.

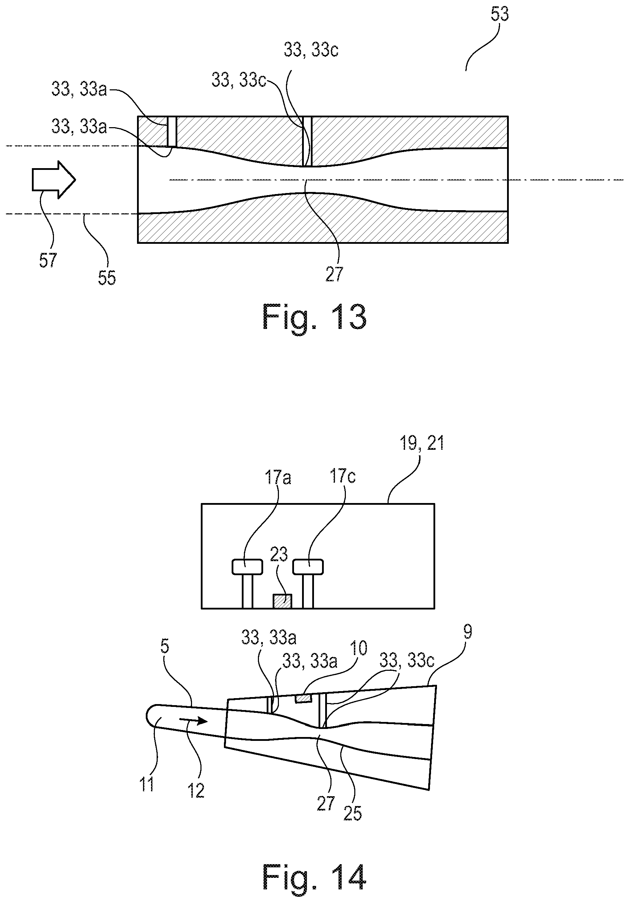

Fig. 13 illustrates a schematic cross-sectional view of a flow restrictor 25 in the form of a venturi 53. The venturi 53 defines a through-going passage 27 with a second minimum cross-sectional area. The venturi 53 comprises barrier elements 33 in the form of flexible membranes and atmospheric air. The barrier elements are arranged to allow measurement of a first pressure by a first pressure gauge 17a in fluid communication with the flow path 12 upstream of the flow restrictor 25 at a first barrier element 33a providing a first pressure communication path and a third pressure by a third pressure gauge 17c at the second minimum cross-sectional area at a third barrier element 33c providing a third pressure communication path. The illustrated venturi 53 may be employed as a flow restrictor 25 in the signal processing device 19 or as a measurement substructure embedded in or integral with the catheter tube 5. In some embodiments, the at first and third pressure gauges 17a and 17c are comprised in the housing 21 of the signal processing device 19 and are configured to interact with the measurement substructure through the barrier elements 33 as illustrated in Fig. 14. The relatively small second minimum cross-sectional area of the through-going passage 27 of the venturi works to increase the fluid velocity therethrough, which in turn results in pressure drop from the first to third pressure gauge 17a, 17c. In practice, a pressure from the first to third pressure gauge also occurs due to the non-zero surface roughness of the venturi 53, while this pressure is smaller for a venturi 53 than for the flow restrictor 25 illustrated in Fig. 2.

Fig. 14 illustrates an embodiment wherein the first and third pressure gauges 17a, 17c are comprised in the housing 21 of the signal processing device 19 and wherein a venturi 53 is embodied as a measurement substructure integrally formed with the non-insertable portion 9 of the catheter tube 5. The connecting portion 10 of the intermittent urinary catheter 3 and the engagement mechanism 23 are embodied as mutually attracting magnets. The non-insertable portion 9 comprises barrier elements 33 in the form of flexible membranes and atmospheric air. The barrier elements are arranged to allow measurement of a first pressure by the first pressure gauge 17a in fluid communication with the flow path 12 upstream of the flow restrictor 25 at the first barrier element 33a and a third pressure by the third pressure gauge 17c at the second minimum cross-sectional area at a third barrier element 33c.

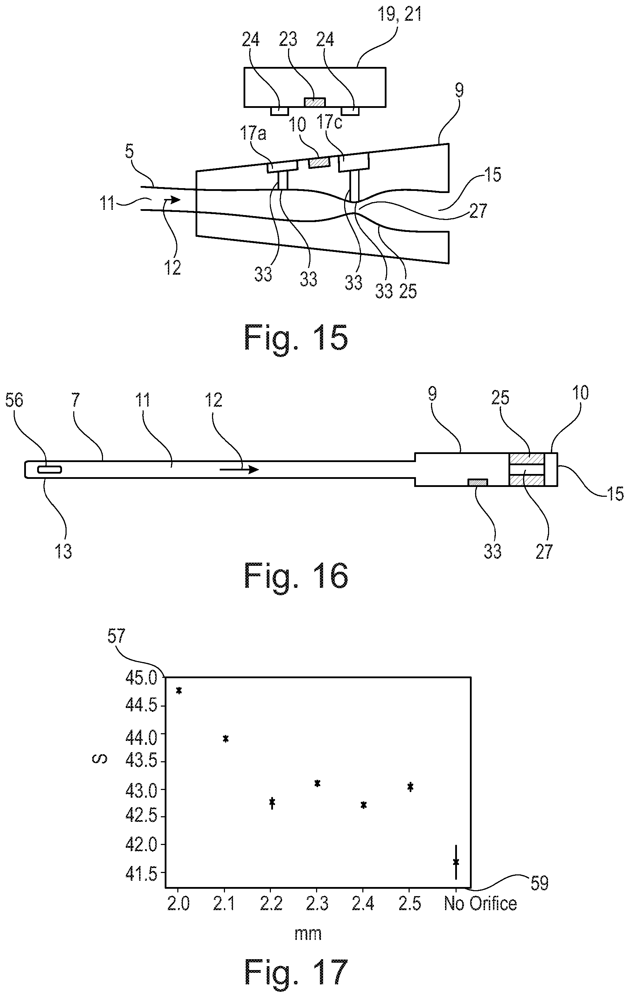

Fig. 15 illustrates an embodiment wherein the venturi 53 is embodied as a measurement substructure integrally formed with the connecting portion 10 of the intermittent urinary catheter 3. In the embodiment of Fig. 15, the first and second pressure gauges 17a, 17c are embedded in the non-insertable portion 9 of the intermittent urinary catheter 3. The interface 24 of the signal processing device 19 for connecting the signal processing device 19 to the sensor elements 17 comprises electrical contacts arranged to contact the first and third pressure gauges 17a, 17c. In one embodiment, the first and second pressure gauges 17a, 17c are provided with electrical power through the interface 24 and a battery of the signal processing device 19.

Fig. 16 illustrates a schematic cross-sectional view of an embodied intermittent urinary catheter 3. The intermittent urinary catheter 3 comprises a catheter tube 5. The catheter tube 5 comprises an insertable portion 7 intended for insertion into a user's urethra, a non-insertable portion 9 not intended for insertion into the user's urethra, and a connecting portion 10 being integral with the non-insertable portion 9. The intermittent urinary catheter 3 further comprises a conduit 11 extending longitudinally within the catheter tube 5. The conduit 11 defines a flow path 12 from a distal insertion end 13 of the catheter 3 to a proximal outlet end 15 thereof. The catheter tube includes an eyelet 56 in the distal insertion end 13. The eyelet 56 allows liquid to enter the conduit 11. The intermittent urinary catheter 3 further comprises a measurement substructure in the form of a flow restrictor 25 embedded in the connecting portion 10. The non-insertable portion 9 comprises a barrier 33 in the form of a flexible barrier allowing measurement of a pressure by a pressure sensor 17 and a measurement substructure in the form of a flow restrictor 25.

Fig. 17 is a plot of measured voiding times of 155 ml of liquid water on the y-axis 57 as a function of the diameter at the second minimum cross-sectional area of the through-going passage 27 of an orifice plate for an embodied intermittent urinary catheterisation assembly 1 on the x-axis 59. The diameter of the internal lumen of the conduit 11 having the first maximum cross-sectional area was 2.6 mm for all plotted measurements. The time on the y-axis is plotted in units of seconds, while the diameter on the x-axis is plotted in units of mm.

**Claims**

1. An intermittent urinary catheterisation assembly (1) for self-catheterisation comprising:

   - an intermittent urinary catheter (3) comprising:

     ○ a catheter tube (5) comprising an insertable portion (7) intended for insertion into a user's urethra, a non-insertable portion (9) not intended for insertion into the user's urethra, and a connecting portion (10) being integral with or mounted to the non-insertable portion (9); and
     ○ a conduit (11) extending longitudinally within the catheter tube (5) and defining at least part of a flow path (12) from a distal insertion end (13) of the catheter (3) to a proximal outlet end (15) thereof;

   - a measuring system (4) for determining at least one fluid parameter in the flow path (12), comprising

     ○ at least one sensor element (17) configured to determine the at least one fluid parameter in the conduit (11) and/or in a space in communication with the conduit (11);
     ○ a signal processing device (19) comprising

       ▪ a housing (21) with an engagement mechanism (23) for detachably securing the signal processing device (19) in relation to the connecting portion (10) of the intermittent urinary catheter (3); and
       ▪ an interface (24) for connecting the signal processing device (19) to the at least one sensor element (17);

   - **characterized in that** the catheter tube (5) is a single-lumen tube, of which the conduit (11) constitutes the sole passage between the distal insertion end (13) and the proximal outlet end (15) of the intermittent urinary catheter (3).

2. An intermittent urinary catheterisation assembly according to claim 1, wherein at least an outer surface of the insertable portion (7) of the catheter tube (5) comprises a hydrophilic surface coating.

3. An intermittent urinary catheterisation assembly according to any of the preceding claims, wherein at least one of the at least one sensor element (17) is comprised in the signal processing device (19).

4. An intermittent urinary catheterisation assembly according to any of the preceding claims, wherein the measuring system (4) comprises a measurement substructure embedded in or integral with the catheter tube (5), the measurement substructure being configured to allow measurement of the at least one fluid parameter in conjunction with the at least one sensor element (17).

5. An intermittent urinary catheterisation assembly according to any of the preceding claims, wherein the measuring system (4) comprises:

   - a first portion (29) comprising the at least one sensor element (17) in the housing (21) of the signal processing device (19), the at least one sensor element (17) being configured to determine the at least one fluid parameter in the conduit (11) and/or in a space in communication with the conduit (11) when the signal processing device (19) is secured in relation to the intermittent urinary catheter (3);
- a second portion (31) for contacting the intermittent urinary catheter (3) and/or its conduit (11);
- a barrier element (33) between the first (29) and the second (31) portions, so as to prevent liquid in the conduit (11) from coming into contact with the at least one sensor element (17).

6. An intermittent urinary catheterisation assembly according to claim 5, wherein the barrier element (33) is embedded in the catheter tube (5).

7. An intermittent urinary catheterisation assembly according to claim 5 or 6, wherein the barrier element (33) comprises a liquid impermeable flexible barrier.

8. An intermittent urinary catheterisation assembly according to any of claims 5-6, wherein the barrier element (33) comprises a gas conduit configured to establish a gaseous buffer between the flow path (12) and the signal processing device (19).

9. An intermittent urinary catheterisation assembly according to any of the preceding claims, wherein at least one of the at least one sensor element (17) is embedded in or integrally formed with the catheter tube (5).

10. An intermittent urinary catheter assembly according to any of the preceding claims, wherein the at least one fluid parameter comprises a pressure, and wherein:

- the assembly (1) comprises a flow restrictor (25) in the flow path (12) or adapted for fluid communication with the flow path (12) and forming a through-going passage (27) configured to restrict the flow therethrough; and wherein
- the measuring system (4) comprises at least one pressure gauge (17a, 17b, 17c, 17d) in fluid communication with the flow path (12).

11. An intermittent urinary catheterisation assembly according to claim 10, wherein:

- the through-going passage (27) defines an inner surface having at least a third liquid coefficient of friction;
- at least part of a perimeter of the flow path (12) upstream of the flow restrictor (25) has a fourth liquid coefficient of friction; and wherein
- the third liquid coefficient of friction is larger than at least the fourth liquid coefficient of friction.

12. An intermittent urinary catheterisation assembly according to claim 10 or 11, wherein:

- the conduit (11) defines an internal lumen having a first maximum cross-sectional area; and wherein
- the through-going passage (27) has a second minimum cross-sectional area.

13. An intermittent urinary catheter assembly according to claim 12, wherein the flow restrictor (25) comprises an orifice plate or orifice tube.

14. An intermittent urinary catheterisation assembly according to any of claims 10-13, wherein the at least one pressure gauge (17a, 17b, 17c, 17d) comprises:

- a first pressure gauge (17a) arranged in the flow path (12) of the conduit (11) or adapted for fluid communication with the flow path upstream of the flow restrictor (25); and
- a third pressure gauge (17c) arranged in the through-going passage (27),

and wherein the signal processing device (19) is configured to determine a fluid flow rate on the basis of a difference between a first pressure determined by the first pressure gauge (17a) and a third pressure determined by the third pressure gauge (17c).

15. An intermittent urinary catheterisation assembly according to any of claims 10-14, wherein the at least one pressure gauge (17a, 17b, 17c, 17d) comprises:

- a first pressure gauge (17a) arranged in the flow path (12) of the conduit (11) or adapted for fluid communication with the flow path upstream of the flow restrictor (25); and
- a second pressure gauge (17b) arranged in the flow path (12) downstream of the flow restrictor (25) or adapted for fluid communication with the flow path downstream of the flow restrictor,

and wherein the signal processing device (19) is configured to determine a fluid flow rate on the basis of a difference between a first pressure determined by the first pressure gauge (17a) and a second pressure determined by the second pressure gauge (17b).

16. An intermittent urinary catheterisation assembly according to any of the preceding claims, wherein the measuring system (4) comprises a valve (37) configured to allow a variable cross-sectional area of fluid flow in fluid communication with the flow path (12).

17. An intermittent urinary catheterisation assembly according to any of claims 14-15 and claim 16, wherein the valve (37) is configured to automatically vary the cross-sectional area for the fluid flow on the basis of a determined fluid flow rate or pressure in the flow path (12).

18. An intermittent urinary catheter (3) for use in an intermittent urinary catheterisation assembly (1) according to any of the preceding claims, comprising

   - a catheter tube (5) comprising an insertable portion (7) intended for insertion into a user's urethra and a non-insertable portion (9) not intended for insertion into the user's urethra, wherein the non-insertable portion (9) defines a connecting portion (10) integral with or mounted to the non-insertable portion (9);
   - a conduit (11) extending longitudinally within the catheter tube (5) and defining at least part of a flow path (12) from a distal insertion end (13) of the intermittent urinary catheter (3) to a proximal outlet end (15) thereof,

   wherein the connecting portion (10) is configured to mate with the engagement mechanism (23) of the assembly (1).

19. An intermittent urinary catheter according to claim 18, wherein the intermittent urinary catheter (3) comprises a measurement substructure embedded in or integral with the catheter tube (5), the measurement substructure being configured to allow measurement of the at least one fluid parameter in conjunction with the at least one sensor element (17) of the assembly (1).

20. An intermittent urinary catheter according to claim 18 or 19, wherein at least one of the at least one sensor element (17) is embedded in or integrally formed with the catheter tube (5).

21. A catheter system comprising a disposable intermittent urinary catheter according to any of claims 18-20 and a package for accommodating the intermittent urinary catheter (3) therein in a sterile condition.

22. A catheter system according to claim 21, wherein at least the insertable portion (7) of the catheter tube (5) comprises a hydrophilic surface coating, and wherein the package accommodates an amount of a liquid swelling medium.

**Patentansprüche**

1. Intermittierende Harnkatheterisierungsanordnung (1) zur Selbstkatheterisierung, umfassend:

   - einen intermittierenden Harnkatheter (3), umfassend:

      ○ ein Katheterrohr (5), umfassend einen einführbaren Abschnitt (7), der zum Einführen in die Urethra eines Anwenders vorgesehen ist, einen nicht-einführbaren Abschnitt (9), der nicht zum Einführen in die Urethra eines Anwenders vorgesehen ist, und einen Verbindungsabschnitt (10), der integral mit dem nicht-einführbaren Abschnitt (9) ist oder darauf montiert ist; und
      ○ eine Rohrleitung (11), die sich in Längsrichtung innerhalb des Katheterrohrs (5) erstreckt und mindestens einen Teil eines Flusspfads (12) von einem distalen Einführende (13) des Katheters (3) zu einem proximalen Auslassende (15) davon definiert;

   - ein Messsystem (4) zum Bestimmen mindestens eines Fluidparameters in dem Flusspfad (12), umfassend

      ○ mindestens ein Sensorelement (17), das ausgestaltet ist, um den mindestens einen Fluidparameter in der Rohrleitung (11) und/oder in einem Raum in Kommunikation mit der Rohrleitung (11) zu bestimmen;
      ○ eine Signalverarbeitungsvorrichtung (19), umfassend

         ■ ein Gehäuse (21) mit einem Eingriffmechanismus (23) zum lösbaren Befestigen der Signalverarbeitungsvorrichtung (19) in Bezug zu dem Verbindungsabschnitt (10) des intermittierenden Harnkatheters (3); und
         ■ eine Schnittstelle (24) zum Verbinden der Signalverarbeitungsvorrichtung (19) mit dem mindestens einen Sensorelement (17);

   - **dadurch gekennzeichnet, dass** das Katheterrohr (5) ein einlumiges Rohr ist, bei dem die Rohrleitung (11) den einzigen Durchlass zwischen dem distalen Einführende (13) und dem proximalen Auslassende (15) des intermittierenden Harnkatheters (3) stellt.

2. Intermittierende Harnkatheterisierungsanordnung nach Anspruch 1, wobei mindestens eine Außenoberfläche des einführbaren Abschnitts (7) des Katheterrohrs (5) eine hydrophile Oberflächenbeschichtung umfasst.

3. Intermittierende Harnkatheterisierungsanordnung nach einem der vorhergehenden Ansprüche, wobei mindestens eines von dem mindestens einen Sensorelement (17) in der Signalverarbeitungsvorrichtung (19) enthalten ist.

4. Intermittierende Harnkatheterisierungsanordnung nach einem der vorhergehenden Ansprüche, wobei das Messsystem (4) eine Messteilstruktur umfasst, die in das Katheterrohr (5) eingebettet ist oder integral damit ist, wobei die Messteilstruktur ausgestaltet ist, um Messung des mindestens einen Fluidparameters in Zusammenhang mit dem mindestens einen Sensorelement (17) zu ermöglichen.

5. Intermittierende Harnkatheterisierungsanordnung nach einem der vorhergehenden Ansprüche, wobei das Messsystem (4) umfasst:

- einen ersten Abschnitt (29), umfassend das mindestens eine Sensorelement (17) in dem Gehäuse (21) der Signalverarbeitungsvorrichtung (19), wobei das mindestens eine Sensorelement (17) ausgestaltet ist, um den mindestens einen Fluidparameter in der Rohrleitung (11) und/oder in einem Raum in Kommunikation mit der Rohrleitung (11) zu bestimmen, wenn die Signalverarbeitungsvorrichtung (19) in Bezug zu dem intermittierenden Harnkatheter (3) befestigt ist;
- einen zweiten Abschnitt (31) zum Kontaktieren des intermittierenden Harnkatheters (3) und/oder dessen Rohrleitung (11);
- ein Barriereelement (33) zwischen dem ersten (29) und dem zweiten (31) Abschnitt, um so zu verhindern, dass Flüssigkeit in der Rohrleitung (11) in Kontakt mit dem mindestens einen Sensorelement (17) kommt.

6. Intermittierende Harnkatheterisierungsanordnung nach Anspruch 5, wobei das Barriereelement (33) in das Katheterrohr (5) eingebettet ist.

7. Intermittierende Harnkatheterisierungsanordnung nach Anspruch 5 oder 6, wobei das Barriereelement (33) eine flüssigkeitsundurchlässige flexible Barriere umfasst.

8. Intermittierende Harnkatheterisierungsanordnung nach einem der Ansprüche 5-6, wobei das Barriereelement (33) eine Gasrohrleitung umfasst, die ausgestaltet ist, um einen gasförmigen Puffer zwischen dem Flusspfad (12) und der Signalverarbeitungsvorrichtung (19) zu erstellen.

9. Intermittierende Harnkatheterisierungsanordnung nach einem der vorhergehenden Ansprüche, wobei mindestens eines von dem mindestens einen Sensorelement (17) in das Katheterrohr (5) eingebettet oder integral damit gebildet ist.

10. Intermittierende Harnkatheterisierungsanordnung nach einem der vorhergehenden Ansprüche, wobei der mindestens eine Fluidparameter einen Druck umfasst, und wobei:

- die Anordnung (1) einen Flussbegrenzer (25) in dem Flusspfad (12) oder eingerichtet zur Fluidkommunikation mit dem Flusspfad (12) umfasst und einen durchgehenden Durchlass (27) bildet, der ausgestaltet ist, um den Fluss durch diesen hindurch zu begrenzen; und wobei
- das Messsystem (4) mindestens einen Druckmesser (17a, 17b, 17c, 17d) in Fluidkommunikation mit dem Flusspfad (12) umfasst.

11. Intermittierende Harnkatheterisierungsanordnung nach Anspruch 10, wobei:

- der durchgehende Durchlass (27) eine Innenoberfläche mit mindestens einem dritten Flüssigkeitsreibungskoeffizienten definiert;
- mindestens ein Teil eines Umfangs des Flusspfads (12) stromaufwärts des Flussbegrenzers (25) einen vierten Flüssigkeitsreibungskoeffizienten aufweist; und wobei
- der dritte Flüssigkeitsreibungskoeffizient größer als mindestens der vierte Flüssigkeitsreibungskoeffizient ist.

12. Intermittierende Harnkatheterisierungsanordnung nach Anspruch 10 oder 11, wobei:

- die Rohrleitung (11) ein Innenlumen mit einer ersten maximalen Querschnittfläche definiert; und wobei
- der durchgehende Durchlass (27) eine zweite minimale Querschnittfläche aufweist.

13. Intermittierende Harnkatheterisierungsanordnung nach Anspruch 12, wobei der Flussbegrenzer (25) eine Drosselblende oder ein Drosselrohr umfasst.

14. Intermittierende Harnkatheterisierungsanordnung nach einem der Ansprüche 10-13, wobei der mindestens eine Druckmesser (17a, 17b, 17c, 17d) umfasst:

- einen ersten Druckmesser (17a), der in dem Flusspfad (12) der Rohrleitung (11) angeordnet oder zur Fluidkommunikation mit dem Flusspfad stromaufwärts des Flussbegrenzers (25) eingerichtet ist; und
- einen dritten Druckmesser (17c), der in dem durchgehenden Durchlass (27) angeordnet ist,

und wobei die Signalverarbeitungsvorrichtung (19) ausgestaltet ist, um eine Fluidflussrate basierend auf einer Differenz zwischen einem von dem ersten Druckmesser (17a) bestimmten ersten Druck und einem durch den dritten Druckmesser (17c) bestimmten dritten Druck zu bestimmen.

15. Intermittierende Harnkatheterisierungsanordnung nach einem der Ansprüche 10-14, wobei der mindestens eine Druckmesser (17a, 17b, 17c, 17d) umfasst:

   - einen ersten Druckmesser (17a), der in dem Flusspfad (12) der Rohrleitung (11) angeordnet oder zur Fluidkommunikation mit dem Flusspfad stromaufwärts des Flussbegrenzers (25) eingerichtet ist; und
   - einen zweiten Druckmesser (17b), der in dem Flusspfad (12) stromabwärts des Flussbegrenzers (25) angeordnet oder zur Fluidkommunikation mit dem Flusspfad stromabwärts des Flussbegrenzers eingerichtet ist,

   und wobei die Signalverarbeitungsvorrichtung (19) ausgestaltet ist, um eine Fluidflussrate basierend auf einer Differenz zwischen einem von dem ersten Druckmesser (17a) bestimmten ersten Druck und einem von dem zweiten Druckmesser (17b) bestimmten zweiten Druck zu bestimmen.

16. Intermittierende Harnkatheterisierungsanordnung nach einem der vorhergehenden Ansprüche, wobei das Messsystem (4) ein Ventil (37) umfasst, das ausgestaltet ist, um eine variable Querschnittfläche des Fluidflusses in Fluidkommunikation mit dem Flusspfad (12) zu ermöglichen.

17. Intermittierende Harnkatheterisierungsanordnung nach einem der Ansprüche 14-15 und Anspruch 16, wobei das Ventil (37) ausgestaltet ist, um die Querschnittfläche für den Fluidfluss basierend auf einer bestimmten Fluidflussrate oder einem bestimmten Druck in dem Flusspfad (12) automatisch zu variieren.

18. Intermittierender Harnkatheter (3) zur Verwendung in einer intermittierenden Harnkatheterisierungsanordnung (1) nach einem der vorhergehenden Ansprüche, umfassend

   - ein Katheterrohr (5), umfassend einen einführbaren Abschnitt (7), der zum Einführen in die Urethra eines Anwenders vorgesehen ist, und einen nicht-einführbaren Abschnitt (9), der nicht zum Einführen in die Urethra eines Anwenders vorgesehen ist, wobei der nichteinführbare Abschnitt (9) einen Verbindungsabschnitt (10) definiert, der integral mit dem nicht-einführbaren Abschnitt (9) ist oder an diesem montiert ist;
   - eine Rohrleitung (11), die sich in Längsrichtung innerhalb eines Katheterrohrs (5) erstreckt und mindestens einen Teil eines Flusspfads (12) von einem distalen Einführende (13) des intermittierenden Harnkatheters (3) zu einem proximalen Auslassende (15) davon definiert,

wobei der Verbindungsabschnitt (10) ausgestaltet ist, um mit dem Eingriffmechanismus (23) der Anordnung (1) gepaart zu werden.

19. Intermittierender Harnkatheter nach Anspruch 18, wobei der intermittierende Harnkatheter (3) eine Messteilstruktur umfasst, die in das Katheterrohr (5) eingebettet oder integral damit ist, wobei die Messteilstruktur ausgestaltet ist, um Messung des mindestens einen Fluidparameters in Zusammenhang mit dem mindestens einen Sensorelement (17) der Anordnung (1) zuzulassen.

20. Intermittierender Harnkatheter nach Anspruch 18 oder 19, wobei mindestens eines von dem mindestens einen Sensorelement (17) in das Katheterrohr (5) eingebettet oder integral damit gebildet ist.

21. Kathetersystem, umfassend einen intermittierenden Einweg-Harnkatheter nach einem der Ansprüche 18-20 und eine Verpackung zur Unterbringung des intermittierenden Harnkatheters (3) darin in einem sterilen Zustand.

22. Kathetersystem nach Anspruch 21, wobei mindestens der einführbare Abschnitt (7) des Katheterrohrs (5) eine hydrophile Oberflächenbeschichtung umfasst, und wobei in der Verpackung eine Menge eines flüssigen Aufquellmediums untergebracht ist.

**Revendications**

1. Ensemble de cathétérisme urinaire intermittent (1) pour autocathétérisme comprenant :

   - un cathéter urinaire intermittent (3) comprenant :

      ◦ un tube de cathéter (5) comprenant une partie insérable (7) destinée à être insérée dans l'urètre d'un utilisateur, une partie non insérable (9) non destinée à être insérée dans l'urètre de l'utilisateur, et une partie de liaison (10) solidaire de la partie non insérable (9) ou montée sur celle-ci ; et
      ◦ un conduit (11) s'étendant longitudinalement à l'intérieur du tube de cathéter (5) et définissant au moins une partie d'une voie d'écoulement (12) d'une extrémité d'insertion distale (13) du cathéter (3) à une extrémité de sortie proximale (15) de celui-ci ;

- un système de mesure (4) pour déterminer au moins un paramètre de fluide dans la voie d'écoulement (12), comprenant

○ au moins un élément capteur (17) configuré pour déterminer le ou les paramètres de fluide dans le conduit (11) et/ou dans un espace en communication avec le conduit (11) ;

○ un dispositif de traitement de signal (19) comprenant

■ un boîtier (21) doté d'un mécanisme d'entrée en prise (23) destiné à fixer de manière amovible le dispositif de traitement de signal (19) par rapport à la partie de liaison (10) du cathéter urinaire intermittent (3) ; et

■ une interface (24) pour relier le dispositif de traitement de signal (19) à l'au moins un élément capteur (17) ;

- **caractérisé en ce que** le tube de cathéter (5) est un tube à lumière unique, dont le conduit (11) constitue le seul passage entre l'extrémité distale d'insertion (13) et l'extrémité proximale de sortie (15) du cathéter urinaire intermittent (3).

2. Ensemble de cathétérisme urinaire intermittent selon la revendication 1, dans lequel au moins une surface externe de la partie insérable (7) du tube de cathéter (5) comprend un revêtement de surface hydrophile.

3. Ensemble de cathétérisme urinaire intermittent selon l'une quelconque des revendications précédentes, dans lequel au moins l'un de l'au moins un élément capteur (17) est compris dans le dispositif de traitement de signal (19).

4. Ensemble de cathétérisme urinaire intermittent selon l'une quelconque des revendications précédentes, dans lequel le système de mesure (4) comprend une sous-structure de mesure noyée dans le tube de cathéter (5) ou solidaire de celui-ci, la sous-structure de mesure étant configurée pour permettre la mesure de l'au moins un paramètre de fluide conjointement avec l'au moins un élément capteur (17).

5. Ensemble de cathétérisme urinaire intermittent selon l'une quelconque des revendications précédentes, dans lequel le système de mesure (4) comprend :

- une première partie (29) comprenant l'au moins un élément capteur (17) dans le boîtier (21) du dispositif de traitement de signal (19), l'au moins un élément capteur (17) étant configuré pour déterminer l'au moins un paramètre de fluide dans le conduit (11) et/ou dans un espace en communication avec le conduit (11) lorsque le dispositif de traitement de signal (19) est fixé par rapport au cathéter urinaire intermittent (3) ;
- une seconde partie (31) destinée à entrer en contact avec le cathéter urinaire intermittent (3) et/ou son conduit (11) ;
- un élément barrière (33) entre les première (29) et seconde (31) parties, de manière à empêcher le liquide dans le conduit (11) d'entrer en contact avec l'au moins un élément capteur (17).

6. Ensemble de cathétérisme urinaire intermittent selon la revendication 5, dans lequel l'élément barrière (33) est noyé dans le tube de cathéter (5).

7. Ensemble de cathétérisme urinaire intermittent selon la revendication 5 ou 6, dans lequel l'élément barrière (33) comprend une barrière flexible imperméable aux liquides.

8. Ensemble de cathétérisme urinaire intermittent selon l'une quelconque des revendications 5 à 6, dans lequel l'élément barrière (33) comprend un conduit de gaz configuré pour établir un tampon gazeux entre la voie d'écoulement (12) et le dispositif de traitement de signal (19).

9. Ensemble de cathétérisme urinaire intermittent selon l'une quelconque des revendications précédentes, dans lequel au moins l'un de l'au moins un élément capteur (17) est noyé dans le tube de cathéter (5) ou formé d'un seul tenant avec celui-ci.

10. Ensemble cathéter urinaire intermittent selon l'une quelconque des revendications précédentes, dans lequel l'au moins un paramètre de fluide comprend une pression, et dans lequel :

- l'ensemble (1) comprend un limiteur d'écoulement (25) dans la voie d'écoulement (12) ou conçu pour une communication fluidique avec la voie d'écoulement (12) et formant un passage traversant (27) configuré pour limiter l'écoulement à travers celui-ci ; et dans lequel
- le système de mesure (4) comprend au moins un manomètre (17a, 17b, 17c, 17d) en communication fluidique avec la voie d'écoulement (12).

11. Ensemble de cathétérisme urinaire intermittent selon la revendication 10, dans lequel :

- le passage traversant (27) définit une surface interne ayant au moins un troisième coefficient de frottement du liquide ;
- au moins une partie d'un périmètre de la voie d'écoulement (12) en amont du limiteur d'écoulement (25) a un quatrième coefficient de frottement de liquide ; et dans lequel

- le troisième coefficient de frottement de liquide est supérieur au moins au quatrième coefficient de frottement de liquide.

12. Ensemble de cathétérisme urinaire intermittent selon la revendication 10 ou 11, dans lequel :

- le conduit (11) définit une lumière interne ayant une première section transversale maximale ; et dans lequel
- le passage traversant (27) présente une seconde section transversale minimale.

13. Ensemble cathéter urinaire intermittent selon la revendication 12, dans lequel le limiteur d'écoulement (25) comprend une plaque à orifice ou un tube à orifice.

14. Ensemble de cathétérisme urinaire intermittent selon l'une quelconque des revendications 10 à 13, dans lequel l'au moins un manomètre (17a, 17b, 17c, 17d) comprend :

- un premier manomètre (17a) disposé dans la voie d'écoulement (12) du conduit (11) ou conçu pour une communication fluidique avec la voie d'écoulement en amont du limiteur d'écoulement (25) ; et
- un troisième manomètre (17c) disposé dans le passage traversant (27),

et dans lequel le dispositif de traitement de signal (19) est configuré pour déterminer un débit de fluide sur la base d'une différence entre une première pression déterminée par le premier manomètre (17a) et une troisième pression déterminée par le troisième manomètre (17c).

15. Ensemble de cathétérisme urinaire intermittent selon l'une quelconque des revendications 10 à 14, dans lequel l'au moins un manomètre (17a, 17b, 17c, 17d) comprend :

- un premier manomètre (17a) disposé dans la voie d'écoulement (12) du conduit (11) ou conçu pour une communication fluidique avec la voie d'écoulement en amont du limiteur d'écoulement (25) ; et
- un deuxième manomètre (17b) disposé dans la voie d'écoulement (12) en aval du limiteur d'écoulement (25) ou conçu pour une communication fluidique avec la voie d'écoulement en aval du limiteur d'écoulement,

et dans lequel le dispositif de traitement de signal (19) est configuré pour déterminer un débit de fluide sur la base d'une différence entre une première pression déterminée par le premier manomètre (17a) et une deuxième pression déterminée par le deuxième manomètre (17b).

16. Ensemble de cathétérisme urinaire intermittent selon l'une quelconque des revendications précédentes, dans lequel le système de mesure (4) comprend une soupape (37) configurée pour permettre une aire de section transversale variable d'écoulement de fluide en communication fluidique avec la voie d'écoulement (12).

17. Ensemble de cathétérisme urinaire intermittent selon l'une quelconque des revendications 14, 15 et 16, dans lequel la soupape (37) est configurée pour faire varier automatiquement l'aire en coupe transversale pour l'écoulement de fluide sur la base d'un débit de fluide déterminé ou d'une pression d'écoulement de fluide déterminée dans la voie d'écoulement (12).

18. Cathéter urinaire intermittent (3) destiné à être utilisé dans un ensemble de cathétérisme urinaire intermittent (1) selon l'une quelconque des revendications précédentes, comprenant

  ◦ un tube de cathéter (5) comprenant une partie insérable (7) destinée à être insérée dans l'urètre d'un utilisateur et une partie non insérable (9) non destinée à être insérée dans l'urètre de l'utilisateur, dans lequel la partie non insérable (9) définit une partie de liaison (10) solidaire de la partie non insérable (9) ou montée sur celle-ci ;
  ◦ un conduit (11) s'étendant longitudinalement à l'intérieur du tube de cathéter (5) et définissant au moins une partie d'une voie d'écoulement (12) d'une extrémité d'insertion distale (13) du cathéter urinaire intermittent (3) à une extrémité de sortie proximale (15) de celui-ci ;

dans lequel la partie de liaison (10) est configurée pour s'accoupler avec le mécanisme d'entrée en prise (23) de l'ensemble (1).

19. Cathéter urinaire intermittent selon la revendication 18, dans lequel le cathéter urinaire intermittent (3) comprend une sous-structure de mesure noyée dans le tube de cathéter (5) ou solidaire de celui-ci, la sous-structure de mesure étant configurée pour permettre la mesure de l'au moins un paramètre de fluide conjointement avec l'au moins un élément capteur (17) de l'ensemble (1).

20. Cathéter urinaire intermittent selon la revendication 18 ou 19, dans lequel au moins l'un de l'au moins un élément capteur (17) est noyé dans le tube de cathéter (5) ou formé d'un seul tenant avec celui-ci.

21. Système de cathéter comprenant un cathéter urinaire intermittent jetable selon l'une quelconque des revendications 18 à 20 et un emballage pour loger le cathéter urinaire intermittent (3) en son sein dans un état stérile.

22. Système de cathéter selon la revendication 21, dans lequel au moins la partie insérable (7) du tube de cathéter (5) comprend un revêtement de surface hydrophile, et dans lequel l'emballage contient une quantité d'un milieu gonflant liquide.

Fig. 1

Fig. 2

Fig. 3

Fig. 4

Fig. 5

Fig. 6

Fig. 7

Fig. 8

Fig. 9

Fig. 10

Fig. 11

Fig. 12

33, 33c

33, 33c

33, 33a

33, 33a

53

57

55

27

## Fig. 13

19, 21

17a    17c

23

5

33, 33a

33, 33a    10    33, 33c    9

11    12

27    25

## Fig. 14

Fig. 15

Fig. 16

Fig. 17

**EP 3 990 084 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20050256447 A
- CN 201782870 U
- CN 107981873 A
- US 20090306539 A1
- CN 103263701 A
- US 5476434 A
- CN 206577223 U